# EUROPEAN PATENT APPLICATION

(11) **EP 4 435 098 A1**
(43) Date of publication of application: **25.09.2024**
(21) Application number: 21963712.1
(22) Date of filing: 15.11.2021
(51) Int. Cl.: C12N 9/12, C12N 15/54, C12P 19/34

(54) **CHIMERIC DNA POLYMERASE AND USE THEREOF**

(71) Applicant: BGI Shenzhen, Shenzhen, Guangdong 518083 (CN)
(72) Inventor: AN, Qun, Shenzhen, Guangdong 518083 (CN); LIN, Qibin, Shenzhen, Guangdong 518083 (CN); GUO, Fei, Shenzhen, Guangdong 518083 (CN); XIE, Qingqing, Shenzhen, Guangdong 518083 (CN); ZHENG, Yue, Shenzhen, Guangdong 518083 (CN); XI, Feng, Shenzhen, Guangdong 518083 (CN); DONG, Yuliang, Shenzhen, Guangdong 518083 (CN); ZHANG, Wenwei, Shenzhen, Guangdong 518083 (CN)
(74) Representative: Schwarz & Partner Patentanwälte GmbH
(86) International application number: PCT/CN2021/130706
(87) International publication number: WO 2023/082266

(57) **Abstract**

Provided is a chimeric DNA polymerase, comprising: a first peptide segment, which has at least 80% homology with at least part of the amino acid sequence in the N-terminal domain of a 9⁰N DNA polymerase; a second peptide segment, which has at least 80% homology with at least part of the amino acid sequence in the exonucleolytic domain of a KOD DNA polymerase; a third peptide segment, which has at least 80% homology with at least some of the amino acids in the N-terminal domain of the 9⁰N DNA polymerase; a fourth peptide segment, which has at least 80% homology with at least some of the amino acids in the palm domain of a KOD DNA polymerase; a fifth peptide segment, which has at least 80% homology with at least some of the amino acids in the finger domain of the Pfu DNA polymerase; a sixth peptide segment, which has at least 80% homology with at least some of the amino acids in the palm domain of the KOD DNA polymerase; and a seventh peptide segment, which has at least 80% homology with at least some of the amino acids in the thumb domain of the 9⁰N DNA polymerase.

## Description

### FIELD

The present disclosure relates to the technical field of biology, and specifically to a chimeric DNA polymerase and use thereof.

### BACKGROUND

DNA polymerase is an enzyme able to synthesize (consequently to replicate), starting from 5' end, a new DNA strand complementary to a sequence of a template strand, with the template strand presenting as a single strand of DNA and four types of deoxyribonucleotide as substrates. DNA polymerase with its polymerization activity enables additions of free nucleotides to 3' end of the newly synthesized strand, leading to an extension of the same in the direction from 5' to 3' end. Furthermore, some of DNA polymerases are of a 3' - 5' exonuclease activity, which can correct errors occurred during synthesis of the new DNA strand. That is, if there is a mismatched base incorporated during PCR amplification, the DNA polymerases with 3' - 5' exonuclease activity would cut it off, reinsert a correct base after removing the mismatched base and continue to replicate, thus ensuring the accuracy of amplification. In general, all of the DNA polymerases belonging to family B are of such a DNA proofreading activity, thus having lower error rates compared with ordinary DNA polymerase (such as Taq DNA polymerase) and being more suitable for experiments requiring high fidelity to PCR, such as gene screening, sequencing, mutation detection, etc. However, the advantages of DNA polymerase for such a proofreading function are counteracted by its relatively low continuous synthesis ability, leading to a reduced yield of DNA amplified products.

With the higher need for the application requirements, in addition to a high amplification yield, there are more requirements put forward for the performance of DNA polymerase, such as faster extension rate, higher amplification specificity, better amplification performance for low amount templates, and better amplification performance for special environments (such as high salt conditions).

There are six DNA polymerase families, i.e. family A, B, C, D, X and Y The thermostable DNA polymerases discovered so far all belong to family A or family B. The DNA polymerases in family A are all derived from eubacteria, for example, Taq (*Thermous aquaticus*), Tth (*Thermous thermophilus*), Tca (*Thermous* caldophilus), Tfl (*Thermousflavus*), Tfi (*Thermous filiformis*) from *Thermus* genus, and Bst (*Bacillus stearothemophilis*) from *Bacillus* genus. The thermostable DNA polymerases in family B are all derived from archaebacteria, such as Tli (*Thermococcus litoralis*), KOD1 (*Thermococcus kodacaraensis*), Tgo (*Thermococcus gorgonarius*) from *Thermococcus* genus, as well as Pfu (*Pyrococcus furiosus*), Pwo (*Pyrococcus woesei*)*,* Pab (*Pyrococcus abyssi*) from *Pyrococcus* genus, etc. The 3' - 5' exonuclease activity of the family B DNA polymerases endows it with the proofreading function.

For the DNA polymerase, the amino acid sequence is the basis of its functional structure. The various functions of the DNA polymerase, such as catalytic activity, proofreading, nucleotide transfer, and substrate binding, have been assigned to various domains individually based on the structure and function analysis thereof. Taken archaebacterial DNA polymerase as an example, the structure of the one is generally divided into five domains, namely, N-terminal domain, exonucleolytic domain, palm domain, finger domain and thumb domain. It is generally believed that the polymerization activity of DNA polymerase is related to the palm, finger and thumb domains. Specifically, the palm domain is considered as the catalytic site of polymerase; the thumb domain interacts with the newly synthesized dsDNA and introduced nucleotides; and the finger domains play a role in template fixation and nucleotide specificity. Furthermore, the exonucleolytic domain relates to the 5' - 3' exonuclease activity, 3' - 5' exonuclease activity, or both, to remove misincorporated bases. Each domain of DNA polymerase cooperates closely with each other to achieve the whole process of DNA replication.

By combining heterologous domains from different DNA polymerases (for example, the polymerase with at least one different functional characteristic), a chimeric DNA polymerase can be formed and may be designed to be derived from any DNA polymerase. When different heterologous domains are fused, special interactions within and between these domains may form specific spatial structures and exhibit corresponding functional characteristics. Appropriate combination of suitable domains presents an enhanced effect on amplification.

The reaction characteristics of PCR and its application requirements determine the following three key properties a DNA polymerase should have, thermal stability, fidelity, and polymerization ability. Moreover, special scenarios (such as rare samples) put forward higher performance requirements for DNA polymerase.

More and more commercial DNA polymerases are engineering protein mutants of naturally existing wild-type DNA polymerases. In the prior art, a variety of functional DNA polymerases and DNA polymerase mutants have been disclosed, many of which have been provided with improved catalytic activity, thermal stability and other properties. However, there are still needs for further improved DNA polymerase mutants with high continuous polymerization capacity, high extension rate, thermal stability, salt resistance, high fidelity and other properties to meet the requirements of DNA amplification, synthesis, detection, sequencing and other important recombinant DNA technologies.

Therefore, the current DNA polymerase remains to be studied.

### SUMMARY

The present disclosure aims to solve at least one of the technical problems in the related art to a certain extent. Therefore, the present disclosure provides a chimeric DNA polymerase and a method for obtaining the same, an isolated nucleic acid, a construct, a recombinant cell or recombinant microorganism, a kit, and use thereof. The chimeric DNA polymerase has the properties of high yield for amplifying products, high specificity, high continuous synthesis ability, high extension rate, thermal stability, strong resistance to salt, high fidelity, etc., meeting the needs of DNA amplification (especially for long fragment amplification), synthesis, detection, sequencing, etc., and having a broad application prospect.

In one aspect, the present disclosure provides in embodiments a chimeric DNA polymerase. According to embodiments of the present disclosure, the chimeric DNA polymerase includes:
a first peptide segment, having at least 80% homology with at least a first part of an amino acid sequence of a N-terminal domain of 9⁰ N DNA polymerase;
a second peptide segment, having at least 80% homology with at least a part of an amino acid sequence of an exonucleolytic domain of KOD DNA polymerase, wherein an N-terminal of the second peptide segment is connected with a C-terminal of the first peptide segment;
a third peptide segment, having at least 80% homology with at least a second part of the amino acid sequence of the N-terminal domain of 9⁰ N DNA polymerase, wherein an N-terminal of the third peptide segment is connected with a C-terminal of the second peptide segment;
a fourth peptide segment, having at least 80% homology with at least a first part of an amino acid sequence of a palm domain of KOD DNA polymerase, wherein an N-terminal of the fourth peptide segment is connected with a C-terminal of the third peptide segment;
a fifth peptide segment, having at least 80% homology with at least a part of an amino acid sequence of a finger domain of Pfu DNA polymerase, wherein an N-terminal of the fifth peptide segment is connected with a C-terminal of the fourth peptide segment;
a sixth peptide segment, having at least 80% homology with at least a second part of the amino acid sequence of the palm domain of KOD DNA polymerase, wherein an N-terminal of the sixth peptide segment is connected with a C-terminal of the fifth peptide segment; and
a seventh peptide segment, having at least 80% homology with at least a part of an amino acid sequence of a thumb domain of 9⁰ N DNA polymerase, wherein an N-terminal of the seventh peptide segment is connected with a C-terminal of the sixth peptide segment.

At present, DNA polymerase that is widely used mainly includes DNA polymerases in family A and family B. The former is represented by Taq DNA polymerase, which has high amplification efficiency but lacks fidelity; while the latter is represented by DNA polymerase such as KOD/Pfu, which has poor performance in presenting high fidelity and continuous synthesis capability meanwhile.

In view of this, in the process of research and development, in order to obtain a DNA polymerase with proofreading function, improved continuous synthesis ability and salt tolerance, DNA polymerases of family A and family B with thermal stability, out of six families, were focused on firstly and candidates for chimerism were selected by analyzing the amplification performance of each DNA polymerase; with polymerase structure analysis, sequence analysis and consideration for the needs of fidelity for amplification, the scope of candidates for chimerism are further narrowed into seven DNA polymerases in the family B DNA polymerase, which were respectively from *Pyrococcus furiosus* (Pfu), *Thermococcus kodacaraensis* (KOD), *Pyrococcus woesei* (Pwo), *Thermococcus 2gorgonarius* (Tgo), *Pyrococcus abyssi* (Pab), *Pyrococcus species* GB-D (Deep vent) and *Thermococcus sp.9⁰* N-7 (9⁰N). Five domains of each of the above seven DNA polymerases in family B may be combined to form different chimeric combinations, which were further analyzed and screened by bioinformatics. Seven candidates were selected for further screening and determining for their expression amount, enzyme activity, thermal stability, salt tolerance, and 3' - 5' exonuclease activity, etc. to obtain the final chimeric DNA polymerase. Therefore, the chimeric DNA polymerase according to embodiment of the present disclosure has the properties of high yield for amplifying products, high specificity, high continuous synthesis ability, high extension rate, thermal stability, strong resistance to salt, high fidelity, etc., meeting the needs of DNA amplification (especially for long fragment amplification), synthesis, detection, sequencing, etc., and having a broad application prospect.

In another aspect, the present disclosure provides in embodiments an isolated nucleic acid. According to embodiments of the present disclosure, the isolated nucleic acid encodes the chimeric DNA polymerase as described above. Accordingly, the isolated nucleic acid according to embodiments of the present disclosure can encode and be used to obtain the chimeric DNA polymerase having the properties of high yield for amplifying products, high specificity, high continuous synthesis ability, high extension rate, thermal stability, strong resistance to salt, high fidelity, etc., therefore meeting the needs of DNA amplification (especially for long fragment amplification), synthesis, detection, sequencing, etc., and having a broad application prospect.

In still another aspect, the present disclosure provides in embodiments a construct. According to embodiments of the present disclosure, the construct includes the isolated nucleic acid as described above. The construct according to embodiments of the present disclosure can be used to express the chimeric DNA polymerase having the properties of high yield for amplifying products, high specificity, high continuous synthesis ability, high extension rate, thermal stability, strong resistance to salt, high fidelity, etc., therefore meeting the needs of DNA amplification, synthesis, detection, sequencing, etc., and having a broad application prospect.

In yet another aspect, the present disclosure provides in embodiments a recombinant cell or a recombinant microorganism. According to embodiments of the present disclosure, the recombinant cell or recombinant microorganism includes the isolated nucleic acid as described above. The recombinant cell or recombinant microorganism according to embodiments of the present disclosure can be used to express the chimeric DNA polymerase having the properties of high yield for amplifying products, high specificity, high continuous synthesis ability, high extension rate, thermal stability, strong resistance to salt, high fidelity, etc., therefore meeting the needs of DNA amplification, synthesis, detection, sequencing, etc., and having a broad application prospect.

In yet another aspect, the present disclosure provides in embodiments a method for obtaining a chimeric DNA polymerase. According to embodiments of the present disclosure, the method includes: cultivating the recombinant cell or the recombinant microorganism as described above in a condition suitable for expressing the chimeric DNA polymerase, so as to obtain the chimeric DNA polymerase. Accordingly, with the method according to embodiments of the present disclosure, the chimeric DNA polymerase having the properties of high yield for amplifying products, high specificity, high continuous synthesis ability, high extension rate, thermal stability, strong resistance to salt, high fidelity, etc. can be obtained, therefore meeting the needs of DNA amplification, synthesis, detection, sequencing, etc., and having a broad application prospect.

In yet another aspect, the present disclosure provides in embodiments a kit. According to embodiments of the present disclosure, the kit includes the chimeric DNA polymerase, the isolated nucleic acid, the construct, or the recombinant cell or recombinant microorganism as described above. Therefore, DNA amplification by using the kit according to embodiments of the present disclosure has the advantages of high yield of amplification product, high amplification accuracy and so on, and is suitable for widespread production and application.

In yet another aspect, the present disclosure provides in embodiments use of the chimeric DNA polymerase, the isolated nucleic acid, the construct, the recombinant cell or recombinant microorganism, or the kit as described above for DNA amplification. Therefore, such DNA amplification has the advantages of high yield of amplification products, high amplification accuracy and so on, and is suitable for widespread production and application.

Additional aspects and advantages of embodiments of the present disclosure will be given in part in the following descriptions, become apparent in part from the following description, be learned from the practice of embodiments of the present disclosure.

### BRIEF DESCRIPTION OF THE DRAWINGS

The above and/or additional aspects and advantages of embodiments of the present disclosure will become apparent and more readily appreciated from the following descriptions made with reference to the drawings, in which:
FIG. 1 is a schematic diagram showing a structure of a chimeric DNA polymerase according to an embodiment of the present disclosure.
FIG. 2 shows an electrophoresis result of a novel chimeric DNA polymerase with purification after expression according to an embodiment of the present disclosure.
FIG. 3 shows an electrophoresis result illustrating amplification performances of the novel chimeric DNA polymerase at different KCl concentrations, according to an embodiment of the present disclosure.
FIG. 4 shows an electrophoresis result of thermo-resistance assay of the novel chimeric DNA polymerase according to an embodiment of the present disclosure.
FIG. 5 shows a result of 3' - 5' exonuclease activity assay of the novel chimeric DNA polymerase according to an embodiment of the present disclosure.

### DETAILED DESCRIPTION

Reference will be made in detail to embodiments of the present disclosure. The embodiments described herein are explanatory, illustrative, and used to generally understand the present disclosure. The embodiments shall not be construed to limit the present disclosure. If the specific technology or conditions are not specified in embodiments, a step will be performed in accordance with the techniques or conditions described in the literature in the art, or in accordance with the product instructions. If the manufacturers of reagents or instruments are not specified, the reagents or instruments may be commercially available.

The embodiments of the present disclosure provide a chimeric DNA polymerase and a method for obtaining the same, an isolated nucleic acid, a construct, a recombinant cell or recombinant microorganism, a kit, and use thereof, which will be described individually in detail below.

### Chimeric DNA polymerase

In one aspect, the present disclosure provides in embodiments a chimeric DNA polymerase. According to the embodiments of the present disclosure, the chimeric DNA polymerase includes: a first peptide segment, having at least 80% homology with at least a first part of an amino acid sequence of a N-terminal domain of 9⁰ N DNA polymerase; a second peptide segment, having at least 80% homology with at least a part of an amino acid sequence of an exonucleolytic domain of KOD DNA polymerase, wherein an N-terminal of the second peptide segment is connected with a C-terminal of the first peptide segment; a third peptide segment, having at least 80% homology with at least a second part of the amino acid sequence of the N-terminal domain of 9⁰ N DNA polymerase, wherein an N-terminal of the third peptide segment is connected with a C-terminal of the second peptide segment; a fourth peptide segment, having at least 80% homology with at least a first part of an amino acid sequence of a palm domain of KOD DNA polymerase, wherein an N-terminal of the fourth peptide segment is connected with a C-terminal of the third peptide segment; a fifth peptide segment, having at least 80% homology with at least a part of an amino acid sequence of a finger domain of Pfu DNA polymerase, wherein an N-terminal of the fifth peptide segment is connected with a C-terminal of the fourth peptide segment; a sixth peptide segment, having at least 80% homology with at least a second part of the amino acid sequence of the palm domain of KOD DNA polymerase, wherein an N-terminal of the sixth peptide segment is connected with a C-terminal of the fifth peptide segment; and a seventh peptide segment, having at least 80% homology with at least a part of an amino acid sequence of a thumb domain of 9⁰ N DNA polymerase, wherein an N-terminal of the seventh peptide segment is connected with a C-terminal of the sixth peptide segment.

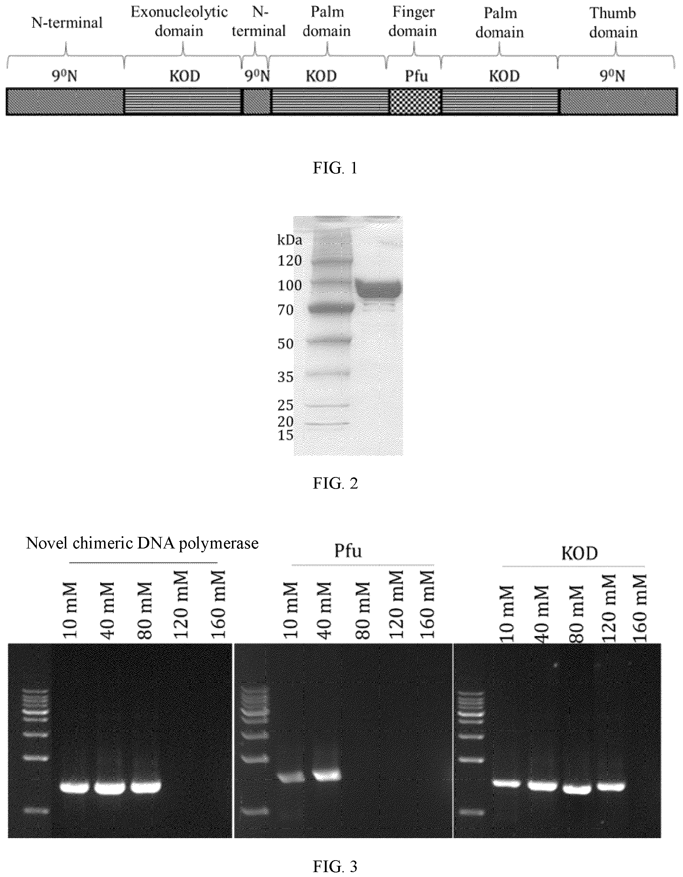

The structure of the chimeric DNA polymerase according to an embodiment of the present disclosure is shown in FIG. 1. The chimeric DNA polymerase in embodiments of the present disclosure has the properties of high yield for amplifying products, high specificity, high continuous synthesis ability, high extension rate, thermal stability, strong resistance to salt, high fidelity, etc., which can meet the needs of DNA amplification (especially for long fragment amplification), synthesis, detection, sequencing, etc., and has a broad application prospect.

The amino acid sequence of 9⁰ N DNA polymerase is as follows:

MILDTDYITENGKPVIRVFKKENGEFKIEYDRTFEPYFYALLKDDSAIEDVKKVTAKR
HGTVVKVKRAEKVQKKFLGRPIEVWKLYFNHPQDVPAIRDRIRAHPAVVDIYEYDIPFAK
RYLIDKGLIPMEGDEELTMLAFDIETLYHEGEEFGTGPILMISYADGSEARVITWKKIDLPY
VDVVSTEKEMIKRFLRVVREKDPDVLITYNGDNFDFAYLKKRCEELGIKFTLGRDGSEPKI
QRMGDRFAVEVKGRIHFDLYPVIRRTINLPTYTLEAVYEAVFGKPKEKVYAEEIAQAWESG
EGLERVARYSMEDAKVTYELGREFFPMEAQLSRLIGQSLWDVSRSSTGNLVEWFLLRKA
YKRNELAPNKPDERELARRRGGYAGGYVKEPERGLWDNIVYLDFRSLYPSIIITHNVSPD
TLNREGCKEYDVAPEVGHKFCKDFPGFIPSLLGDLLEERQKIKRKMKATVDPLEKKLLDY
RQRAIKILANSFYGYYGYAKARWYCKECAESVTAWGREYIEMVIRELEEKFGFKVLYAD
TDGLHATIPGADAETVKKKAKEFLKYINPKLPGLLELEYEGFYVRGFFVTKKKYAVIDEE
GKITTRGLEIVRRDWSEIAKETQARVLEAILKHGDVEEAVRIVKEVTEKLSKYEVPPEKLV
IHEQITRDLRDYKATGPHVAVAKRLAARGVKIRPGTVISYIVLKGSGRIGDRAIPADEFDPT
KHRYDAEYYIENQVLPAVERILKAFGYRKEDLRYQKTKQVGLGAWLKVKGKK(SEQ ID
NO: 19).

The amino acid sequence of KOD DNA polymerase is as follows:

MILDTDYITEDGKPVIRIFKKENGEFKIEYDRTFEPYFYALLKDDSAIEEVKKITAERH
GTVVTVKRVEKVQKKFLGRPVEVWKLYFTHPQDVPAIRDKIREHPAVIDIYEYDIPFAKRY
LIDKGLVPMEGDEELKMLAFDIETLYHEGEEFAEGPILMISYADEEGARVITWKNVDLPYV
DVVSTEREMIKRFLRVVKEKDPDVLITYNGDNFDFAYLKKRCEKLGINFALGRDGSEPKI
QRMGDRFAVEVKGRIHFDLYPVIRRTINLPTYTLEAVYEAVFGQPKEKVYAEEITTAWETG
ENLERVARYSMEDAKVTYELGKEFLPMEAQLSRLVGQSLWDVSRSSTGNLVEWFLLRKA
YERNELAPNKPDEKELARRRQSYEGGYVKEPERGLWENIVYLDFRSIAPSIIITHNVSPDT
LNREGCKEYDVAPQVGHRFCKDFPGFIPSLLGDLLEERQKIKKKMKATIDPIERKLLDYR
QRAIKILANSYYGYYGYARARWYCKECAESVTAWGREYITMTIKEIEEKYGFKVIYSDTD
GFFATIPGADAETVKKKAMEFLKYINAKLPGALELEYEGFYKRGFFVTKKKYAVIDEEGK
ITTRGLEIVRRDWSEIAKETQARVLEALLKDGDVEKAVRIVKEVTEKLSKYEVPPEKLVIH
EQITRDLKDYKATGPHVAVAKRLAARGVKIRPGTVISYIVLKGSGRIGDRAIPFDEFDPTK

HKYDAEYYIENQVLPAVERILRAFGYRKEDLRYQKTRQVGLSAWLKPKGT(SEQ ID NO:
20).

The amino acid sequence of Pfu DNA polymerase is as follows:

MILDVDYITEEGKPVIRLFKKENGKFKIEHDRTFRPYIYALLRDDSKIEEVKKITGERH
GKIVRIVDVEKVEKKFLGKPITVWKLYLEHPQDVPTLREKVREHPAVVDIFEYDIPFAKRY
LIDKGLIPMEGEEELKILAFDIETLYHEGEEFGKGPIIMISYADENEARVITWKNIDLPYVES
VSTEKEMIKRFLRIIREKDPDIIVTYNGDSFDFPYLAKRAEKLGIKLTIGRDGSEPKMQRIG
DMTAVEVKGRIHFDLYHVIRTTINLPTYTLEAVYEAIFGKPKEKVYADEIAKAWESGENLE
RVAKYSMEDAKATYELGKEFLPMEIQLSRLVGQPLWDVSRSSTGNLVEWFLLRKAYERN
EVAPNKPSEEEYQRRLRESYTGGFVKEPEKGLWENIVYLDYKSLYPSIIITHNVSPDTLNLE
GCKNYDIAPQVGHKFCKDIPGFIPSLLGHLLEERQKIKTKMKETQDPIEKILLDYRQKAIK
LLANSFYGYYGYAKARWYCKECAESVTAWGRKYIELVWKELEEKFGFKVLYIDTDGLYA
TIPGGESEEIKKKALEFVKYINSKLPGLLELEYEGFYKRGFFVTKKRYAVIDEEGKVITRGL
EIVRRDWSEIAKETQARVLETILKHGDVEEAVRIVKEVIQKLANYEIPPEKLAIYEQITRPL
HEYKAIGPHVAVAKKLAAKGVKIKPGMVIGYIVLRGDGPISNRAILAEEYDPKKHKYDAE
YYIENQVLPAVLRILEGFGYRKEDLRYQKTRQVGLTSWLNIKKS(SEQ ID NO: 21).

According to embodiments of the present disclosure, the chimeric DNA polymerase as described above may also have the following additional technical features.

According to embodiments of the present disclosure, the first peptide segment has at least 80% homology with an amino acid sequence at positions 1 to 390 of the amino acid sequence for 9⁰ N DNA polymerase.

According to embodiments of the present disclosure, the second peptide segment has at least 80% homology with an amino acid sequence at positions 391 to 1014 of the amino acid sequence for KOD DNA polymerase.

According to embodiments of the present disclosure, the third peptide segment has at least 80% homology with an amino acid sequence at positions 1015 to 1116 of the amino acid sequence for 9⁰ N DNA polymerase.

According to embodiments of the present disclosure, the fourth peptide segment has at least 80% homology with an amino acid sequence at positions 1117 to 1341 of the amino acid sequence for KOD DNA polymerase.

According to embodiments of the present disclosure, the fifth peptide segment has at least 80% homology with an amino acid sequence at positions 1345 to 1500 of the amino acid sequence for Pfu DNA polymerase.

According to embodiments of the present disclosure, the sixth peptide segment has at least 80% homology with an amino acid sequence at positions 1498 to 1770 of the amino acid sequence for KOD DNA polymerase.

According to embodiments of the present disclosure, the seventh peptide has at least 80% homology with an amino acid sequence at positions 1771 to 2328 of the amino acid sequence for 9⁰ N DNA polymerase.

According to embodiments of the present disclosure, the chimeric DNA polymerase is of an amino acid sequence as depicted in SEQ ID NO: 1:

MASAILDTDYITENGKPVIRVFKKENGEFKIEYDRTFEPYFYALLKDDSAIEDVKKVT
AKRHGTVVKVKRAEKVQKKFLGRPIEVWKLYFNHPQDVPAIRDRIRAHPAVVDIYEYDIP
FAKRYLIDKGLIPMEGDEELKMLAFDIETLYHEGEEFAEGPILMISYADEEGARVITWKNV
DLPYVDVVSTEREMIKRFLRVVKEKDPDVLITYNGDNFDFAYLKKRCEKLGINFALGRDG
SEPKIQRMGDRFAVEVKGRIHFDLYPVIRRTINLPTYTLEAVYEAVFGQPKEKVYAEEITTA
WETGENLERVARYSMEDAKVTYELGKEFLPMEAQLSRLVGQSLWDVSRSSTGNLVEWF
LLRKAYKRNELAPNKPDEKELARRRQSYEGGYVKEPERGLWENIVYLDFRSIAPSIIITHN
VSPDTLNREGCKEYDVAPQVGHRFCKDFPGFIPSLLGHLLEERQKIKTKMKETQDPIEKIL
LDYRQKAIKLLANSFYGYYGYARARWYCKECAESVTAWGREYITMTIKEIEEKYGFKVI
YSDTDGFFATIPGADAETVKKKAMEFLKYINAKLPGALELEYEGFYKRGFFVTKKKYAVI
DEEGKITTRGLEIVRRDWSEIAKETQARVLEAILKHGDVEEAVRIVKEVTEKLSKYEVPPE
KLVIHEQITRDLRDYKATGPHVAVAKRLAARGVKIRPGTVISYIVLKGSGRIGDRAIPADEF
DPTKHRYDAEYYIENQVLPAVERILKAFGYRKEDLRYQKTKQVGLGAWLKVKGKK

According to embodiments of the present disclosure, the chimeric DNA polymerase has at least one mutation selected from the following mutations, compared with the amino acid sequence as depicted in SEQ ID NO: 1: M162I, I540V, A598T, H728Q, F37Y, D48V, R100H, Y221N, K243N, Q245L, I271T, E296V, N307S, F751Y, L758Q, V766I, E154A, L44Q, Y149H, R196C, F217H, D346H, D715E, F155A, Q94H and Q94L.

On the basis of the chimeric DNA polymerase as described above, modifications and screenings were performed on the same to further improve its PCR performance, such as amplification yield, faster extension rate, ability to amplify low-quality templates and amplification specificity. Taken the chimeric DNA polymerase as a template, a mutant library was constructed by error-prone PCR and expressed (as described in Example 2 and Example 3). During screening the mutants, mutation sites that affect and improve the performance of the chimeric polymerase were determined by comparing the expression amount, heat resistance, salt tolerance, amplification of low input templates (as described in Example 4), amplification ability for long fragments (as described in Example 5), amplification specificity of target fragments at low annealing temperature (as described in Example 6), etc. The performance of the chimeric DNA polymerase thereby can be further improved.

According to embodiments of the present disclosure, the chimeric DNA polymerase has a group of mutations selected from the following groups: group I: M162I, I540V, A598T and H728Q; group II: F37Y, D48V, R100H, Y221N, K243N, Q245L, I271T, E296V, N307S, F751Y, L758Q, V766I and E154A; group III: F37Y, L44Q, D48V, R100H, Y149H, K243N, Q245L, I271T, E296V, N307S, F751Y, L758Q, V766I and E154A; group IV: F37Y, D48V, R100H, R196C, F217H, Y221N, K243N, Q245L, I271T, E296V, N307S, D346H, F751Y, L758Q, V766I and E154A; group V: F37Y, D48V, Q94L, R100H, Y221N, K243N, Q245L, I271T, E296V, N307S, F751Y, L758Q, V766I and E154A; group VI: E296V, N307S, F751Y, L758Q and E154A; group VII: F37Y, D48V, Q94H, R100H, Y221N, K243N, Q245L, I271T, E296V, N307S, D715E, H728Q, F751Y, L758Q, V766I and E154A; and group VIII: F37Y, D48V, Q94L, R100H, F155A, Y221N, K243N, Q245L, I271T, E296V, N307S, F751Y, L758Q, V766I and E154A. The chimeric DNA polymerase with mutation combinations set forth in the above eight groups has higher yield of amplification products and compatibility with broader PCR applications, such as amplifications with low amount of templates, amplifications for long fragments and amplifications for complex templates, etc., and thus can be widely used for DNA amplification, synthesis, detection, sequencing and other important recombinant DNA technologies.

According to embodiments of the present disclosure, the chimeric DNA polymerase is of an amino acid sequence as depicted in any one of SEQ ID NOs: 2-9.

According to embodiments of the present disclosure, an amino acid sequence of a chimeric DNA polymerase 1-3 having the mutations of group I is as follows:

MASAILDTDYITENGKPVIRVFKKENGEFKIEYDRTFEPYFYALLKDDSAIEDVKKVT
AKRHGTVVKVKRAEKVQKKFLGRPIEVWKLYFNHPQDVPAIRDRIRAHPAVVDIYEYDIP
FAKRYLIDKGLIPMEGDEELKMLAFDIETLYHEGEEFAEGPILIISYADEEGARVITWKNVD
LPYVDVVSTEREMIKRFLRVVKEKDPDVLITYNGDNFDFAYLKKRCEKLGINFALGRDGS
EPKIQRMGDRFAVEVKGRIHFDLYPVIRRTINLPTYTLEAVYEAVFGQPKEKVYAEEITTA
WETGENLERVARYSMEDAKVTYELGKEFLPMEAQLSRLVGQSLWDVSRSSTGNLVEWF

LLRKAYKRNELAPNKPDEKELARRRQSYEGGYVKEPERGLWENIVYLDFRSIAPSIIITHN
VSPDTLNREGCKEYDVAPQVGHRFCKDFPGFIPSLLGHLLEERQKIKTKMKETQDPIEKIL
LDYRQKAIKLLANSFYGYYGYARARWYCKECAESVTAWGREYITMTIKEIEEKYGFKVV
YSDTDGFFATIPGADAETVKKKAMEFLKYINAKLPGALELEYEGFYKRGFFVTKKKYTVI
DEEGKITTRGLEIVRRDWSEIAKETQARVLEAILKHGDVEEAVRIVKEVTEKLSKYEVPPE
KLVIHEQITRDLRDYKATGPHVAVAKRLAARGVKIRPGTVISYIVLKGSGRIGDRAIPADEF
DPTKQRYDAEYYIENQVLPAVERILKAFGYRKEDLRYQKTKQVGLGAWLKVKGKK
(SEQ ID NO: 2).

According to embodiments of the present disclosure, an amino acid sequence of a chimeric DNA polymerase E5 having the mutations of group II is as follows:

MASAILDTDYITENGKPVIRVFKKENGEFKIEYDRTYEPYFYALLKDVSAIEDVKKVT
AKRHGTVVKVKRAEKVQKKFLGRPIEVWKLYFNHPQDVPAIHDRIRAHPAVVDIYEYDIP
FAKRYLIDKGLIPMEGDEELKMLAFDIETLYHEGEAFAEGPILMISYADEEGARVITWKNV
DLPYVDVVSTEREMIKRFLRVVKEKDPDVLITYNGDNFDFANLKKRCEKLGINFALGRD
GSEPNILRMGDRFAVEVKGRIHFDLYPVIRRTTNLPTYTLEAVYEAVFGQPKEKVYAVEITT
AWETGESLERVARYSMEDAKVTYELGKEFLPMEAQLSRLVGQSLWDVSRSSTGNLVEWF
LLRKAYKRNELAPNKPDEKELARRRQSYEGGYVKEPERGLWENIVYLDFRSIAPSIIITHN
VSPDTLNREGCKEYDVAPQVGHRFCKDFPGFIPSLLGHLLEERQKIKTKMKETQDPIEKIL
LDYRQKAIKLLANSFYGYYGYARARWYCKECAESVTAWGREYITMTIKEIEEKYGFKVI
YSDTDGFFATIPGADAETVKKKAMEFLKYINAKLPGALELEYEGFYKRGFFVTKKKYAVI
DEEGKITTRGLEIVRRDWSEIAKETQARVLEAILKHGDVEEAVRIVKEVTEKLSKYEVPPE
KLVIHEQITRDLRDYKATGPHVAVAKRLAARGVKIRPGTVISYIVLKGSGRIGDRAIPADEF
DPTKHRYDAEYYIENQVLPAVERILKAYGYRKEDQRYQKTKQIGLGAWLKVKGKK (SEQ
ID NO: 3).

According to embodiments of the present disclosure, an amino acid sequence of a chimeric DNA polymerase E8 having the mutations of group III is as follows:

MASAILDTDYITENGKPVIRVFKKENGEFKIEYDRTYEPYFYAQLKDVSAIEDVKKV
TAKRHGTVVKVKRAEKVQKKFLGRPIEVWKLYFNHPQDVPAIHDRIRAHPAVVDIYEYDI
PFAKRYLIDKGLIPMEGDEELKMLAFDIETLHHEGEAFAEGPILMISYADEEGARVITWKN
VDLPYVDVVSTEREMIKRFLRVVKEKDPDVLITYNGDNFDFAYLKKRCEKLGINFALGRD
GSEPNILRMGDRFAVEVKGRIHFDLYPVIRRTTNLPTYTLEAVYEAVFGQPKEKVYAVEITT
AWETGESLERVARYSMEDAKVTYELGKEFLPMEAQLSRLVGQSLWDVSRSSTGNLVEWF

LLRKAYKRNELAPNKPDEKELARRRQSYEGGYVKEPERGLWENIVYLDFRSIAPSIIITHN
VSPDTLNREGCKEYDVAPQVGHRFCKDFPGFIPSLLGHLLEERQKIKTKMKETQDPIEKIL
LDYRQKAIKLLANSFYGYYGYARARWYCKECAESVTAWGREYITMTIKEIEEKYGFKVI
YSDTDGFFATIPGADAETVKKKAMEFLKYINAKLPGALELEYEGFYKRGFFVTKKKYAVI
DEEGKITTRGLEIVRRDWSEIAKETQARVLEAILKHGDVEEAVRIVKEVTEKLSKYEVPPE
KLVIHEQITRDLRDYKATGPHVAVAKRLAARGVKIRPGTVISYIVLKGSGRIGDRAIPADEF
DPTKHRYDAEYYIENQVLPAVERILKAYGYRKEDQRYQKTKQIGLGAWLKVKGKK (SEQ
ID NO: 4).

According to embodiments of the present disclosure, an amino acid sequence of a chimeric DNA polymerase A4-2 having the mutations of group IV is as follows:

MASAILDTDYITENGKPVIRVFKKENGEFKIEYDRTYEPYFYALLKDVSAIEDVKKVT
AKRHGTVVKVKRAEKVQKKFLGRPIEVWKLYFNHPQDVPAIHDRIRAHPAVVDIYEYDIP
FAKRYLIDKGLIPMEGDEELKMLAFDIETLYHEGEAFAEGPILMISYADEEGARVITWKNV
DLPYVDVVSTEREMIKCFLRVVKEKDPDVLITYNGDNHDFANLKKRCEKLGINFALGRD
GSEPNILRMGDRFAVEVKGRIHFDLYPVIRRTTNLPTYTLEAVYEAVFGQPKEKVYAVEITT
AWETGESLERVARYSMEDAKVTYELGKEFLPMEAQLSRLVGQSLWHVSRSSTGNLVEWF
LLRKAYKRNELAPNKPDEKELARRRQSYEGGYVKEPERGLWENIVYLDFRSIAPSIIITHN
VSPDTLNREGCKEYDVAPQVGHRFCKDFPGFIPSLLGHLLEERQKIKTKMKETQDPIEKIL
LDYRQKAIKLLANSFYGYYGYARARWYCKECAESVTAWGREYITMTIKEIEEKYGFKVI
YSDTDGFFATIPGADAETVKKKAMEFLKYINAKLPGALELEYEGFYKRGFFVTKKKYAVI
DEEGKITTRGLEIVRRDWSEIAKETQARVLEAILKHGDVEEAVRIVKEVTEKLSKYEVPPE
KLVIHEQITRDLRDYKATGPHVAVAKRLAARGVKIRPGTVISYIVLKGSGRIGDRAIPADEF
DPTKHRYDAEYYIENQVLPAVERILKAYGYRKEDQRYQKTKQIGLGAWLKVKGKK (SEQ
ID NO: 5).

According to embodiments of the present disclosure, an amino acid sequence of a chimeric DNA polymerase QDC4 having the mutations of group V is as follows:

MASAILDTDYITENGKPVIRVFKKENGEFKIEYDRTYEPYFYALLKDVSAIEDVKKVT
AKRHGTVVKVKRAEKVQKKFLGRPIEVWKLYFNHPLDVPAIHDRIRAHPAVVDIYEYDIP
FAKRYLIDKGLIPMEGDEELKMLAFDIETLYHEGEAFAEGPILMISYADEEGARVITWKNV
DLPYVDVVSTEREMIKRFLRVVKEKDPDVLITYNGDNFDFANLKKRCEKLGINFALGRD
GSEPNILRMGDRFAVEVKGRIHFDLYPVIRRTTNLPTYTLEAVYEAVFGQPKEKVYAVEITT
AWETGESLERVARYSMEDAKVTYELGKEFLPMEAQLSRLVGQSLWDVSRSSTGNLVEWF

LLRKAYKRNELAPNKPDEKELARRRQSYEGGYVKEPERGLWENIVYLDFRSIAPSIIITHN
VSPDTLNREGCKEYDVAPQVGHRFCKDFPGFIPSLLGHLLEERQKIKTKMKETQDPIEKIL
LDYRQKAIKLLANSFYGYYGYARARWYCKECAESVTAWGREYITMTIKEIEEKYGFKVI
YSDTDGFFATIPGADAETVKKKAMEFLKYINAKLPGALELEYEGFYKRGFFVTKKKYAVI
DEEGKITTRGLEIVRRDWSEIAKETQARVLEAILKHGDVEEAVRIVKEVTEKLSKYEVPPE
KLVIHEQITRDLRDYKATGPHVAVAKRLAARGVKIRPGTVISYIVLKGSGRIGDRAIPADEF
DPTKHRYDAEYYIENQVLPAVERILKAYGYRKEDQRYQKTKQIGLGAWLKVKGKK (SEQ
ID NO: 6).

According to embodiments of the present disclosure, an amino acid sequence of a chimeric DNA polymerase 1-4 having the mutations of group VI is as follows:

MASAILDTDYITENGKPVIRVFKKENGEFKIEYDRTFEPYFYALLKDDSAIEDVKKVT
AKRHGTVVKVKRAEKVQKKFLGRPIEVWKLYFNHPQDVPAIRDRIRAHPAVVDIYEYDIP
FAKRYLIDKGLIPMEGDEELKMLAFDIETLYHEGEAFAEGPILMISYADEEGARVITWKNV
DLPYVDVVSTEREMIKRFLRVVKEKDPDVLITYNGDNFDFAYLKKRCEKLGINFALGRDG
SEPKIQRMGDRFAVEVKGRIHFDLYPVIRRTINLPTYTLEAVYEAVFGQPKEKVYAVEITTA
WETGESLERVARYSMEDAKVTYELGKEFLPMEAQLSRLVGQSLWDVSRSSTGNLVEWFL
LRKAYKRNELAPNKPDEKELARRRQSYEGGYVKEPERGLWENIVYLDFRSIAPSIIITHNV
SPDTLNREGCKEYDVAPQVGHRFCKDFPGFIPSLLGHLLEERQKIKTKMKETQDPIEKILL
DYRQKAIKLLANSFYGYYGYARARWYCKECAESVTAWGREYITMTIKEIEEKYGFKVIY
SDTDGFFATIPGADAETVKKKAMEFLKYINAKLPGALELEYEGFYKRGFFVTKKKYAVID
EEGKITTRGLEIVRRDWSEIAKETQARVLEAILKHGDVEEAVRIVKEVTEKLSKYEVPPEK
LVIHEQITRDLRDYKATGPHVAVAKRLAARGVKIRPGTVISYIVLKGSGRIGDRAIPADEFD
PTKHRYDAEYYIENQVLPAVERILKAYGYRKEDQRYQKTKQVGLGAWLKVKGKK (SEQ
ID NO: 7).

According to embodiments of the present disclosure, an amino acid sequence of a chimeric DNA polymerase QAA1 having the mutations of group VII is as follows:

MASAILDTDYITENGKPVIRVFKKENGEFKIEYDRTYEPYFYALLKDVSAIEDVKKVT
AKRHGTVVKVKRAEKVQKKFLGRPIEVWKLYFNHPHDVPAIHDRIRAHPAVVDIYEYDIP
FAKRYLIDKGLIPMEGDEELKMLAFDIETLYHEGEAFAEGPILMISYADEEGARVITWKNV
DLPYVDVVSTEREMIKRFLRVVKEKDPDVLITYNGDNFDFANLKKRCEKLGINFALGRD
GSEPNILRMGDRFAVEVKGRIHFDLYPVIRRTTNLPTYTLEAVYEAVFGQPKEKVYAVEITT
AWETGESLERVARYSMEDAKVTYELGKEFLPMEAQLSRLVGQSLWDVSRSSTGNLVEWF

LLRKAYKRNELAPNKPDEKELARRRQSYEGGYVKEPERGLWENIVYLDFRSIAPSIIITHN
VSPDTLNREGCKEYDVAPQVGHRFCKDFPGFIPSLLGHLLEERQKIKTKMKETQDPIEKIL
LDYRQKAIKLLANSFYGYYGYARARWYCKECAESVTAWGREYITMTIKEIEEKYGFKVI
YSDTDGFFATIPGADAETVKKKAMEFLKYINAKLPGALELEYEGFYKRGFFVTKKKYAVI
DEEGKITTRGLEIVRRDWSEIAKETQARVLEAILKHGDVEEAVRIVKEVTEKLSKYEVPPE
KLVIHEQITRDLRDYKATGPHVAVAKRLAARGVKIRPGTVISYIVLKGSGRIGERAIPADEF
DPTKQRYDAEYYIENQVLPAVERILKAYGYRKEDQRYQKTKQIGLGAWLKVKGKK (SEQ
ID NO: 8).

According to embodiments of the present disclosure, an amino acid sequence of a chimeric DNA polymerase QAA3 having the mutations of group VIII is as follows:

MASAILDTDYITENGKPVIRVFKKENGEFKIEYDRTYEPYFYALLKDVSAIEDVKKVT
AKRHGTVVKVKRAEKVQKKFLGRPIEVWKLYFNHPLDVPAIHDRIRAHPAVVDIYEYDIP
FAKRYLIDKGLIPMEGDEELKMLAFDIETLYHEGEAAAEGPILMISYADEEGARVITWKNV
DLPYVDVVSTEREMIKRFLRVVKEKDPDVLITYNGDNFDFANLKKRCEKLGINFALGRD
GSEPNILRMGDRFAVEVKGRIHFDLYPVIRRTTNLPTYTLEAVYEAVFGQPKEKVYAVEITT
AWETGESLERVARYSMEDAKVTYELGKEFLPMEAQLSRLVGQSLWDVSRSSTGNLVEWF
LLRKAYKRNELAPNKPDEKELARRRQSYEGGYVKEPERGLWENIVYLDFRSIAPSIIITHN
VSPDTLNREGCKEYDVAPQVGHRFCKDFPGFIPSLLGHLLEERQKIKTKMKETQDPIEKIL
LDYRQKAIKLLANSFYGYYGYARARWYCKECAESVTAWGREYITMTIKEIEEKYGFKVI
YSDTDGFFATIPGADAETVKKKAMEFLKYINAKLPGALELEYEGFYKRGFFVTKKKYAVI
DEEGKITTRGLEIVRRDWSEIAKETQARVLEAILKHGDVEEAVRIVKEVTEKLSKYEVPPE
KLVIHEQITRDLRDYKATGPHVAVAKRLAARGVKIRPGTVISYIVLKGSGRIGDRAIPADEF
DPTKHRYDAEYYIENQVLPAVERILKAYGYRKEDQRYQKTKQIGLGAWLKVKGKK (SEQ
ID NO: 9).

In another aspect, the present disclosure provides in embodiments an isolated nucleic acid. According to embodiments of the present disclosure, the isolated nucleic acid encodes the chimeric DNA polymerase as described above. Accordingly, the isolated nucleic acid according to embodiments of the present disclosure encodes and can be used to obtain the chimeric DNA polymerase having the properties of high yield for amplifying products, high specificity, high continuous synthesis ability, high extension rate, thermal stability, strong resistance to salt, high fidelity, etc., therefore meeting the needs of DNA amplification (especially for long fragment amplification), synthesis, detection, sequencing, etc., and having a broad application prospect.

According to embodiments of the present disclosure, the isolated nucleic acid has the nucleotide sequence as depicted in SEQ ID NO: 10 as follows:

ATGGCGAGCGCGATTCTGGACACTGATTACATTACCGAAAACGGTAAACCGGTTA
TCCGCGTGTTCAAGAAAGAGAATGGTGAGTTCAAAATCGAGTACGATCGCACGTTTG
AACCGTACTTCTATGCTCTGCTGAAAGACGATTCTGCGATTGAAGATGTGAAAAAAGT
GACGGCGAAACGTCACGGCACCGTGGTTAAGGTGAAACGTGCGGAGAAAGTGCAAA
AGAAATTCCTGGGCCGTCCGATCGAAGTTTGGAAGCTGTACTTTAACCACCCACAAG
ACGTCCCGGCGATTCGTGACCGCATCCGTGCGCACCCGGCTGTGGTTGACATCTATGA
GTACGATATTCCGTTCGCTAAGAGATACTTGATTGACAAGGGTCTGATCCCTATGGAAG
GTGACGAAGAACTGAAGATGCTGGCGTTCGACATCGAAACTCTGTACCACGAGGGTG
AAGAGTTTGCCGAGGGTCCGATCTTGATGATTTCCTACGCGGACGAAGAGGGCGCAC
GTGTTATCACGTGGAAAAATGTTGATCTGCCGTATGTTGACGTCGTAAGCACCGAGCG
TGAGATGATCAAACGTTTTCTGCGCGTTGTTAAAGAAAAAGATCCTGACGTGCTGATC
ACCTACAACGGTGACAATTTCGATTTCGCGTACCTGAAGAAACGTTGCGAAAAACTG
GGTATTAACTTCGCGCTGGGTCGCGATGGCTCTGAACCGAAGATCCAGCGCATGGGTG
ATCGTTTTGCGGTCGAGGTGAAGGGTCGCATTCATTTCGACCTGTACCCGGTGATTCG
TCGTACCATCAACTTGCCGACTTACACCCTGGAAGCCGTCTATGAAGCTGTATTTGGTC
AACCGAAAGAAAAAGTGTACGCTGAGGAAATTACGACGGCGTGGGAAACCGGTGAG
AACCTGGAGCGCGTTGCACGTTATTCTATGGAGGACGCGAAAGTTACCTACGAACTGG
GTAAAGAGTTCCTGCCGATGGAGGCCCAACTGTCCCGTCTGGTGGGCCAAAGCCTGT
GGGACGTCAGCCGTTCGTCCACCGGCAACTTGGTTGAATGGTTCCTGCTGCGTAAGG
CATACAAGCGTAACGAACTGGCGCCGAATAAGCCGGACGAGAAAGAATTGGCGCGTC
GCCGCCAGAGCTATGAGGGTGGTTATGTCAAAGAACCGGAGCGCGGCTTGTGGGAGA
ACATCGTCTATTTGGATTTTCGTAGCATTGCACCGAGCATCATTATCACGCATAATGTGA
GCCCGGATACGTTGAATCGTGAGGGCTGTAAGGAATACGACGTGGCGCCTCAGGTTG
GCCACCGTTTCTGCAAGGACTTTCCGGGCTTTATACCAAGTCTCTTGGGACATTTGTTA
GAGGAAAGACAAAAGATTAAGACAAAAATGAAGGAAACTCAAGATCCTATAGAAAA
AATACTCCTTGACTATAGACAAAAAGCGATAAAACTCTTAGCAAATTCTTTCTACGGAT
ATTATGGCTATGCGCGTGCGCGCTGGTATTGCAAAGAGTGTGCCGAGAGCGTGACCGC
TTGGGGTCGTGAGTACATTACCATGACGATCAAAGAGATTGAAGAGAAATACGGCTTT
AAGGTTATCTATAGCGACACCGACGGTTTCTTTGCAACTATCCCTGGCGCAGACGCAG
AAACCGTTAAGAAAAAGGCAATGGAGTTTCTGAAGTATATCAACGCGAAGTTGCCAG

GCGCCCTGGAACTGGAGTACGAGGGCTTCTACAAGCGTGGCTTTTTCGTGACGAAGA
AGAAGTACGCGGTCATTGACGAAGAGGGCAAGATTACGACCCGTGGTCTGGAAATTG
TTCGCCGTGACTGGTCCGAGATTGCGAAAGAAACCCAGGCGAGAGTGCTGGAAGCG
ATTCTGAAGCATGGTGATGTCGAGGAAGCCGTGCGTATCGTTAAAGAAGTGACGGAG
AAGTTGAGCAAGTACGAAGTCCCACCGGAGAAACTGGTGATTCATGAGCAGATCACG
CGCGATTTACGTGACTATAAAGCAACCGGTCCGCATGTTGCCGTGGCAAAGCGTCTGG
CTGCGCGTGGCGTTAAGATCCGTCCGGGCACGGTTATTAGCTACATTGTGTTGAAAGG
TAGCGGTCGTATTGGCGACCGCGCCATTCCGGCCGACGAGTTCGATCCGACCAAGCAC
CGCTACGATGCAGAGTATTACATCGAGAACCAAGTGCTGCCGGCTGTAGAGCGTATTC
TGAAGGCATTCGGTTATCGTAAAGAAGATCTGCGCTATCAAAAGACGAAACAAGTTG
GCCTGGGTGCGTGGCTGAAGGTCAAGGGCAAGAAATAA (SEQ ID NO: 10).

According to embodiments of the present disclosure, a nucleotide sequence of 9⁰ N DNA polymerase is as follows:

ATGATTCTGGACACTGATTACATTACCGAAAACGGTAAACCGGTTATCCGCGTGTT
CAAGAAAGAGAATGGTGAGTTCAAAATCGAGTACGATCGCACGTTTGAACCGTACTT
CTATGCTCTGCTGAAAGACGATTCTGCGATTGAAGATGTGAAAAAAGTGACGGCGAA
ACGTCACGGCACCGTGGTTAAGGTGAAACGTGCGGAGAAAGTGCAAAAGAAATTCC
TGGGCCGTCCGATCGAAGTTTGGAAGCTGTACTTTAACCACCCACAAGACGTCCCGG
CGATTCGTGACCGCATCCGTGCGCACCCGGCTGTGGTTGACATCTATGAGTACGATATT
CCGTTCGCTAAGAGATACTTGATTGACAAGGGTCTGATCCCTATGGAAGGCGACGAAG
AACTGACCATGCTGGCCTTCGATATCGAGACGTTGTATCACGAGGGCGAAGAGTTTGG
CACCGGCCCAATCCTGATGATTAGCTATGCCGACGGTTCCGAAGCGCGTGTGATCACC
TGGAAGAAAATTGATCTGCCGTACGTCGATGTGGTGAGCACGGAAAAAGAAATGATC
AAACGTTTTCTGCGTGTGGTCCGTGAGAAAGATCCGGATGTCCTGATTACGTATAACG
GTGACAATTTTGATTTTGCGTACCTGAAAAAGCGCTGCGAGGAACTGGGTATCAAGTT
CACGCTGGGTCGTGATGGTAGCGAGCCGAAGATTCAGCGTATGGGTGACCGTTTTGCA
GTTGAGGTGAAGGGTCGCATTCACTTCGACCTGTACCCGGTTATTCGCCGCACCATCA
ACTTGCCTACCTACACCCTGGAAGCGGTCTATGAAGCTGTCTTTGGCAAACCGAAAGA
GAAAGTTTACGCGGAAGAGATCGCGCAGGCGTGGGAGAGCGGTGAGGGTCTGGAAC
GTGTTGCCCGCTACAGCATGGAAGATGCGAAGGTGACTTATGAGTTGGGTCGCGAGTT
TTTCCCGATGGAAGCACAGCTGAGCCGTCTGATCGGCCAAAGCCTGTGGGACGTCAG
CCGTTCGTCCACCGGCAACTTGGTTGAATGGTTCCTGCTGCGTAAGGCATACAAGCGT

AACGAACTGGCGCCGAATAAGCCGGACGAGCGTGAGCTGGCCCGTCGCCGTGGTGGT
TATGCCGGTGGCTATGTTAAAGAGCCGGAGCGCGGTCTGTGGGACAATATCGTGTATC
TGGACTTCCGCTCCCTGTATCCGAGCATCATTATCACCCACAATGTTAGCCCGGATACT
TTAAACCGCGAGGGTTGTAAAGAGTACGACGTGGCGCCTGAGGTCGGCCACAAGTTT
TGCAAAGATTTCCCGGGCTTCATCCCAAGCCTGCTGGGCGATCTGCTGGAGGAACGTC
AGAAGATCAAACGCAAAATGAAAGCAACGGTTGATCCGCTGGAGAAAAAGCTGCTG
GATTATCGTCAGCGCGCAATTAAGATCCTGGCGAATAGCTTTTATGGTTACTACGGTTAT
GCCAAAGCGCGTTGGTACTGTAAAGAATGCGCTGAGTCTGTCACCGCGTGGGGCCGT
GAGTACATCGAAATGGTTATCCGTGAGCTCGAAGAGAAATTCGGTTTTAAGGTTCTGT
ATGCCGACACCGACGGTCTGCACGCGACCATCCCGGGTGCAGACGCCGAAACCGTCA
AGAAGAAAGCAAAAGAATTTCTGAAATACATTAATCCGAAATTGCCGGGTCTGTTGGA
GTTGGAGTATGAGGGTTTCTACGTTCGTGGCTTCTTTGTTACCAAGAAGAAGTACGCG
GTCATTGACGAAGAGGGCAAGATTACGACCCGTGGTCTGGAAATTGTTCGCCGTGAC
TGGTCCGAGATTGCGAAAGAAACCCAGGCGAGAGTGCTGGAAGCGATTCTGAAGCAT
GGTGATGTCGAGGAAGCCGTGCGTATCGTTAAAGAAGTGACGGAGAAGTTGAGCAAG
TACGAAGTCCCACCGGAGAAACTGGTGATTCATGAGCAGATCACGCGCGATTTACGTG
ACTATAAAGCAACCGGTCCGCATGTTGCCGTGGCAAAGCGTCTGGCTGCGCGTGGCG
TTAAGATCCGTCCGGGCACGGTTATTAGCTACATTGTGTTGAAAGGTAGCGGTCGTATT
GGCGACCGCGCCATTCCGGCCGACGAGTTCGATCCGACCAAGCACCGCTACGATGCA
GAGTATTACATCGAGAACCAAGTGCTGCCGGCTGTAGAGCGTATTCTGAAGGCATTCG
GTTATCGTAAAGAAGATCTGCGCTATCAAAAGACGAAACAAGTTGGCCTGGGTGCGT
GGCTGAAGGTCAAGGGCAAGAAATAA (SEQ ID NO: 22).

According to embodiments of the present disclosure, a nucleotide sequence of Pfu DNA polymerase is as follows:

ATGATTTTAGATGTGGATTACATAACTGAAGAAGGAAAACCTGTTATTAGGCTATT
CAAAAAAGAGAACGGAAAATTTAAGATAGAGCATGATAGAACTTTTAGACCATACATT
TACGCTCTTCTCAGGGATGATTCAAAGATTGAAGAAGTTAAGAAAATAACGGGGGAA
AGGCATGGAAAGATTGTGAGAATTGTTGATGTAGAGAAGGTTGAGAAAAAGTTTCTC
GGCAAGCCTATTACCGTGTGGAAACTTTATTTGGAACATCCCCAAGATGTTCCCACTTT
AAGAGAAAAAGTTAGAGAACATCCAGCAGTTGTGGACATCTTCGAATACGATATTCCA
TTTGCAAAGAGATACCTCATCGACAAAGGCCTAATACCAATGGAGGGGGAAGAAGAG
CTAAAGATTCTTGCCTTCGATATAGAAACCCTCTATCACGAAGGAGAAGAGTTTGGAA

AAGGCCCAATTATAATGATTAGTTATGCAGATGAAAATGAAGCAAGGGTGATTACTTGG
AAAAACATAGATCTTCCATACGTTGAGTCAGTATCAACCGAGAAAGAGATGATAAAGA
GATTTCTCAGGATTATCAGGGAGAAGGATCCTGACATTATAGTTACTTATAATGGAGAC
TCATTCGACTTCCCATATTTAGCGAAAAGGGCAGAAAAACTTGGGATTAAATTAACCA
TTGGAAGAGATGGAAGCGAGCCCAAGATGCAGAGAATAGGCGATATGACGGCTGTAG
AAGTCAAGGGAAGAATACATTTCGACTTGTATCATGTAATAAGGACAACAATAAATCT
CCCAACATACACACTAGAGGCTGTATATGAAGCAATTTTTGGAAAGCCAAAGGAGAA
GGTATACGCCGACGAGATAGCAAAAGCCTGGGAAAGTGGAGAGAACCTTGAGAGAG
TTGCCAAATACTCGATGGAAGATGCAAAGGCAACTTATGAACTCGGGAAAGAATTCCT
TCCAATGGAAATTCAGCTTTCAAGATTAGTTGGACAACCTTTATGGGATGTTTCAAGGT
CAAGCACAGGGAACCTTGTAGAGTGGTTCTTACTTAGGAAAGCCTACGAAAGAAACG
AAGTAGCTCCAAACAAGCCAAGTGAAGAGGAGTATCAAAGAAGGCTCAGGGAGAGC
TACACAGGTGGATTCGTTAAAGAGCCAGAAAAGGGGTTGTGGGAAAACATAGTATAC
CTAGATTACAAATCACTATATCCCTCGATTATAATTACCCACAATGTTTCTCCCGATACTC
TAAATCTTGAGGGATGCAAGAACTATGATATCGCTCCTCAAGTAGGCCACAAGTTCTG
CAAGGACATCCCTGGTTTTATACCAAGTCTCTTGGGACATTTGTTAGAGGAAAGACAA
AAGATTAAGACAAAAATGAAGGAAACTCAAGATCCTATAGAAAAAATACTCCTTGACT
ATAGACAAAAAGCGATAAAACTCTTAGCAAATTCTTTCTACGGATATTATGGCTATGCA
AAAGCAAGATGGTACTGTAAGGAGTGTGCTGAGAGCGTTACTGCCTGGGGAAGAAAG
TACATCGAGTTAGTATGGAAGGAGCTCGAAGAAAAGTTTGGATTTAAAGTCCTCTACA
TTGACACTGATGGTCTCTATGCAACTATCCCAGGAGGAGAAAGTGAGGAAATAAAGA
AAAAGGCTCTAGAATTTGTAAAATACATAAATTCAAAGCTCCCTGGACTGCTAGAGCT
TGAATATGAAGGGTTTTATAAGAGGGGATTCTTCGTTACGAAGAAGAGGTATGCAGTA
ATAGATGAAGAAGGAAAAGTCATTACTCGTGGTTTAGAGATAGTTAGGAGAGATTGGA
GTGAAATTGCAAAAGAAACTCAAGCTAGAGTTTTGGAGACAATACTAAAACACGGAG
ATGTTGAAGAAGCTGTGAGAATAGTAAAAGAAGTAATACAAAAGCTTGCCAATTATGA
AATTCCACCAGAGAAGCTCGCAATATATGAGCAGATAACAAGACCATTACATGAGTAT
AAGGCGATAGGTCCTCACGTAGCTGTTGCAAAGAAACTAGCTGCTAAAGGAGTTAAA
ATAAAGCCAGGAATGGTAATTGGATACATAGTACTTAGAGGCGATGGTCCAATTAGCA
ATAGGGCAATTCTAGCTGAGGAATACGATCCCAAAAAGCACAAGTATGACGCAGAATA
TTACATTGAGAACCAGGTTCTTCCAGCGGTACTTAGGATATTGGAGGGATTTGGATACA
GAAAGGAAGACCTCAGATACCAAAAGACAAGACAAGTCGGCCTAACTTCCTGGCTTA

ACATTAAAAAATCCTGA (SEQ ID NO: 23).

According to embodiments of the present disclosure, a nucleotide sequence of KOD DNA polymerase is as follows:

ATGATTCTGGACACCGATTACATCACCGAAGATGGCAAGCCAGTTATCCGCATTTT
CAAAAAAGAGAATGGTGAATTCAAGATCGAATATGATCGTACCTTCGAGCCGTACTTC
TATGCTCTGCTGAAAGACGATAGCGCGATTGAGGAGGTCAAGAAAATCACCGCGGAG
CGTCACGGTACGGTTGTTACCGTGAAACGCGTGGAGAAAGTCCAGAAGAAATTTCTG
GGTCGCCCGGTTGAAGTGTGGAAGCTGTACTTTACGCATCCGCAAGATGTTCCGGCGA
TTCGCGATAAGATTCGTGAGCACCCGGCAGTCATTGACATCTACGAGTATGACATTCCG
TTCGCCAAGCGTTATCTGATCGATAAGGGTCTGGTCCCGATGGAGGGTGACGAAGAAC
TGAAGATGCTGGCGTTCGACATCGAAACTCTGTACCACGAGGGTGAAGAGTTTGCCG
AGGGTCCGATCTTGATGATTTCCTACGCGGACGAAGAGGGCGCACGTGTTATCACGTG
GAAAAATGTTGATCTGCCGTATGTTGACGTCGTAAGCACCGAGCGTGAGATGATCAAA
CGTTTTCTGCGCGTTGTTAAAGAAAAAGATCCTGACGTGCTGATCACCTACAACGGTG
ACAATTTCGATTTCGCGTACCTGAAGAAACGTTGCGAAAAACTGGGTATTAACTTCGC
GCTGGGTCGCGATGGCTCTGAACCGAAGATCCAGCGCATGGGTGATCGTTTTGCGGTC
GAGGTGAAGGGTCGCATTCATTTCGACCTGTACCCGGTGATTCGTCGTACCATCAACT
TGCCGACTTACACCCTGGAAGCCGTCTATGAAGCTGTATTTGGTCAACCGAAAGAAAA
AGTGTACGCTGAGGAAATTACGACGGCGTGGGAAACCGGTGAGAACCTGGAGCGCG
TTGCACGTTATTCTATGGAGGACGCGAAAGTTACCTACGAACTGGGTAAAGAGTTCCT
GCCGATGGAGGCCCAACTGTCCCGTCTGGTGGGCCAAAGCCTGTGGGACGTTAGCCG
CAGCAGCACCGGTAACTTAGTTGAATGGTTCTTGCTGCGTAAGGCATACGAACGCAAT
GAGCTGGCGCCGAACAAACCGGACGAGAAAGAATTGGCGCGTCGCCGCCAGAGCTA
TGAGGGTGGTTATGTCAAAGAACCGGAGCGCGGCTTGTGGGAGAACATCGTCTATTT
GGATTTTCGTAGCATTGCACCGAGCATCATTATCACGCATAATGTGAGCCCGGATACGT
TGAATCGTGAGGGCTGTAAGGAATACGACGTGGCGCCTCAGGTTGGCCACCGTTTCT
GCAAGGACTTTCCGGGCTTTATCCCGAGCCTGCTGGGTGATTTGCTGGAGGAACGTCA
GAAAATCAAGAAGAAGATGAAAGCAACCATTGATCCGATCGAGCGCAAATTACTGGA
CTACCGTCAACGTGCCATCAAGATCCTGGCGAATTCGTATTATGGTTACTATGGCTACG
CGCGTGCGCGCTGGTATTGCAAAGAGTGTGCCGAGAGCGTGACCGCTTGGGGTCGTG
AGTACATTACCATGACGATCAAAGAGATTGAAGAGAAATACGGCTTTAAGGTTATCTAT
AGCGACACCGACGGTTTCTTTGCAACTATCCCTGGCGCAGACGCAGAAACCGTTAAG

AAAAAGGCAATGGAGTTTCTGAAGTATATCAACGCGAAGTTGCCAGGCGCCCTGGAA
CTGGAGTACGAGGGCTTCTACAAGCGTGGCTTTTTCGTGACGAAAAAGAAATACGCT
GTTATTGATGAAGAGGGCAAGATCACGACCCGTGGCCTGGAAATTGTGCGCCGTGATT
GGAGCGAAATTGCAAAAGAAACGCAAGCGCGTGTGCTGGAAGCGCTGCTGAAGGAC
GGCGACGTCGAAAAAGCTGTGCGTATTGTTAAAGAGGTCACCGAGAAGCTGAGCAA
ATACGAGGTCCCGCCAGAGAAATTGGTGATTCACGAACAGATTACGCGTGACCTGAA
AGACTATAAGGCCACCGGTCCGCATGTCGCAGTGGCGAAGCGCCTGGCGGCTCGCGG
TGTGAAGATCCGTCCGGGTACCGTCATTAGCTATATCGTGCTGAAGGGCAGCGGTCGT
ATCGGCGACCGTGCGATTCCGTTCGACGAATTTGATCCGACCAAACACAAATATGATG
CGGAATACTATATTGAGAACCAAGTGCTGCCAGCCGTTGAGCGTATTCTGCGCGCCTT
CGGTTACCGCAAGGAAGATCTGCGTTACCAGAAAACTCGTCAGGTCGGTCTGTCCGC
ATGGCTGAAACCGAAGGGCACCTGA (SEQ ID NO: 24).

According to embodiments of the present disclosure, the isolated nucleic acid is of a nucleotide sequence as depicted in any one of SEQ ID NOs: 11-18.

According to embodiments of the present disclosure, a nucleotide sequence of the mutant 1-3 is as follows:

ATGGCGAGCGCGATTCTGGACACTGATTACATTACCGAAAACGGTAAACCGGTTA
TCCGCGTGTTCAAGAAAGAGAATGGTGAGTTCAAAATCGAGTACGATCGCACGTTTG
AACCGTACTTCTATGCTCTGCTGAAAGACGATTCTGCGATTGAAGATGTGAAAAAAGT
GACGGCGAAACGTCACGGCACCGTGGTTAAGGTGAAACGTGCGGAGAAAGTGCAAA
AGAAATTCCTGGGCCGTCCGATCGAAGTTTGGAAGCTGTACTTTAACCACCCACAAG
ACGTCCCGGCGATTCGTGACCGCATCCGTGCGCACCCGGCTGTGGTTGACATCTATGA
GTACGATATTCCGTTCGCTAAGAGATACTTGATTGACAAGGGTCTGATCCCTATGGAAG
GTGACGAAGAACTGAAGATGCTGGCGTTCGACATCGAAACTCTGTACCACGAGGGTG
AAGAGTTTGCCGAGGGTCCGATCTTGATCATTTCCTACGCGGACGAAGAGGGCGCAC
GTGTTATCACGTGGAAAAATGTTGATCTGCCGTATGTTGACGTCGTAAGCACCGAGCG
TGAGATGATCAAACGTTTTCTGCGCGTTGTTAAAGAAAAAGATCCTGACGTGCTGATC
ACCTACAACGGTGACAATTTCGATTTCGCGTACCTGAAGAAACGTTGCGAAAAACTG
GGTATTAACTTCGCGCTGGGTCGCGATGGCTCTGAACCGAAGATCCAGCGCATGGGTG
ATCGTTTTGCGGTCGAGGTGAAGGGTCGCATTCATTTCGACCTGTACCCGGTGATTCG
TCGTACCATCAACTTGCCGACTTACACCCTGGAAGCCGTCTATGAAGCTGTATTTGGTC
AACCGAAAGAAAAAGTGTACGCTGAGGAAATTACGACGGCGTGGGAAACCGGTGAG

AACCTGGAGCGCGTTGCACGTTATTCTATGGAGGACGCGAAAGTTACCTACGAACTGG
GTAAAGAGTTCCTGCCGATGGAGGCCCAACTGTCCCGTCTGGTGGGCCAAAGCCTGT
GGGACGTCAGCCGTTCGTCCACCGGCAACTTGGTTGAATGGTTCCTGCTGCGTAAGG
CATACAAGCGTAACGAACTGGCGCCGAATAAGCCGGACGAGAAAGAATTGGCGCGTC
GCCGCCAGAGCTATGAGGGTGGTTATGTCAAAGAACCGGAGCGCGGCTTGTGGGAGA
ACATCGTCTATTTGGATTTTCGTAGCATTGCACCGAGCATCATTATCACGCATAATGTGA
GCCCGGATACGTTGAATCGTGAGGGCTGTAAGGAATACGACGTGGCGCCTCAGGTTG
GCCACCGTTTCTGCAAGGACTTTCCGGGCTTTATACCAAGTCTCTTGGGACATTTGTTA
GAGGAAAGACAAAAGATTAAGACAAAAATGAAGGAAACTCAAGATCCTATAGAAAA
AATACTCCTTGACTATAGACAAAAAGCGATAAAACTCTTAGCAAATTCTTTCTACGGAT
ATTATGGCTATGCGCGTGCGCGCTGGTATTGCAAAGAGTGTGCCGAGAGCGTGACCGC
TTGGGGTCGTGAGTACATTACCATGACGATCAAAGAGATTGAAGAGAAATACGGCTTT
AAGGTTGTTTATAGCGACACCGACGGTTTCTTTGCAACTATCCCTGGCGCAGACGCAG
AAACCGTTAAGAAAAAGGCAATGGAGTTTCTGAAGTATATCAACGCGAAGTTGCCAG
GCGCCCTGGAACTGGAGTACGAGGGCTTCTACAAGCGTGGCTTTTTCGTGACGAAGA
AGAAGTACACGGTCATTGACGAAGAGGGCAAGATTACGACCCGTGGTCTGGAAATTG
TTCGCCGTGACTGGTCCGAGATTGCGAAAGAAACCCAGGCGAGAGTGCTGGAAGCG
ATTCTGAAGCATGGTGATGTCGAGGAAGCCGTGCGTATCGTTAAAGAAGTGACGGAG
AAGTTGAGCAAGTACGAAGTCCCACCGGAGAAACTGGTGATTCATGAGCAGATCACG
CGCGATTTACGTGACTATAAAGCAACCGGTCCGCATGTTGCCGTGGCAAAGCGTCTGG
CTGCGCGTGGCGTTAAGATCCGTCCGGGCACGGTTATTAGCTACATTGTGTTGAAAGG
TAGCGGTCGTATTGGCGACCGCGCCATTCCGGCCGACGAGTTCGATCCGACCAAGCA
ACGCTACGATGCAGAGTATTACATCGAGAACCAAGTGCTGCCGGCTGTAGAGCGTATT
CTGAAGGCATTCGGTTATCGTAAAGAAGATCTGCGCTATCAAAAGACGAAACAAGTTG
GCCTGGGTGCGTGGCTGAAGGTCAAGGGCAAGAAATAA (SEQ ID NO: 11).

According to embodiments of the present disclosure, a nucleotide sequence of the mutant E5 is as follows:

ATGGCGAGCGCGATTCTGGACACTGATTACATTACCGAAAACGGTAAACCGGTTA
TCCGCGTGTTCAAGAAAGAGAATGGTGAGTTCAAAATCGAGTACGATCGCACGTATG
AACCGTACTTCTATGCTCTGCTGAAAGACGTTTCTGCGATTGAAGATGTGAAAAAAGT
GACGGCGAAACGTCACGGCACCGTGGTTAAGGTGAAACGTGCGGAGAAAGTGCAAA
AGAAATTCCTGGGCCGTCCGATCGAAGTTTGGAAGCTGTACTTTAACCACCCACAAG

ACGTCCCGGCGATTCATGACCGCATCCGTGCGCACCCGGCTGTGGTTGACATCTATGA
GTACGATATTCCGTTCGCTAAGAGATACTTGATTGACAAGGGTCTGATCCCTATGGAAG
GTGACGAAGAACTGAAGATGCTGGCGTTCGACATCGAAACTCTGTACCACGAGGGTG
AAGCGTTTGCCGAGGGTCCGATCTTGATGATTTCCTACGCGGACGAAGAGGGCGCAC
GTGTTATCACGTGGAAAAATGTTGATCTGCCGTATGTTGACGTCGTAAGCACCGAGCG
TGAGATGATCAAACGTTTTCTGCGCGTTGTTAAAGAAAAAGATCCTGACGTGCTGATC
ACCTACAACGGTGACAATTTCGATTTCGCGAATCTGAAGAAACGTTGCGAAAAACTG
GGTATTAACTTCGCGCTGGGTCGCGATGGCTCTGAACCGAATATCCTGCGCATGGGTG
ATCGTTTTGCGGTCGAGGTGAAGGGTCGCATTCATTTCGACCTGTACCCGGTGATTCG
TCGTACCACCAACTTGCCGACTTACACCCTGGAAGCCGTCTATGAAGCTGTATTTGGT
CAACCGAAAGAAAAAGTGTACGCTGTGGAAATTACGACGGCGTGGGAAACCGGTGA
GAGCCTGGAGCGCGTTGCACGTTATTCTATGGAGGACGCGAAAGTTACCTACGAACTG
GGTAAAGAGTTCCTGCCGATGGAGGCCCAACTGTCCCGTCTGGTGGGCCAAAGCCTG
TGGGACGTCAGCCGTTCGTCCACCGGCAACTTGGTTGAATGGTTCCTGCTGCGTAAGG
CATACAAGCGTAACGAACTGGCGCCGAATAAGCCGGACGAGAAAGAATTGGCGCGTC
GCCGCCAGAGCTATGAGGGTGGTTATGTCAAAGAACCGGAGCGCGGCTTGTGGAGA
ACATCGTCTATTTGGATTTTCGTAGCATTGCACCGAGCATCATTATCACGCATAATGTGA
GCCCGGATACGTTGAATCGTGAGGGCTGTAAGGAATACGACGTGGCGCCTCAGGTTG
GCCACCGTTTCTGCAAGGACTTTCCGGGCTTTATACCAAGTCTCTTGGGACATTTGTTA
GAGGAAAGACAAAAGATTAAGACAAAAATGAAGGAAACTCAAGATCCTATAGAAAA
AATACTCCTTGACTATAGACAAAAAGCGATAAAACTCTTAGCAAATTCTTTCTACGGAT
ATTATGGCTATGCGCGTGCGCGCTGGTATTGCAAAGAGTGTGCCGAGAGCGTGACCGC
TTGGGGTCGTGAGTACATTACCATGACGATCAAAGAGATTGAAGAGAAATACGGCTTT
AAGGTTATCTATAGCGACACCGACGGTTTCTTTGCAACTATCCCTGGCGCAGACGCAG
AAACCGTTAAGAAAAAGGCAATGGAGTTTCTGAAGTATATCAACGCGAAGTTGCCAG
GCGCCCTGGAACTGGAGTACGAGGGCTTCTACAAGCGTGGCTTTTTCGTGACGAAGA
AGAAGTACGCGGTCATTGACGAAGAGGGCAAGATTACGACCCGTGGTCTGGAAATTG
TTCGCCGTGACTGGTCCGAGATTGCGAAAGAAACCCAGGCGAGAGTGCTGGAAGCG
ATTCTGAAGCATGGTGATGTCGAGGAAGCCGTGCGTATCGTTAAAGAAGTGACGGAG
AAGTTGAGCAAGTACGAAGTCCCACCGGAGAAACTGGTGATTCATGAGCAGATCACG
CGCGATTTACGTGACTATAAAGCAACCGGTCCGCATGTTGCCGTGGCAAAGCGTCTGG
CTGCGCGTGGCGTTAAGATCCGTCCGGGCACGGTTATTAGCTACATTGTGTTGAAAGG

TAGCGGTCGTATTGGCGACCGCGCCATTCCGGCCGACGAGTTCGATCCGACCAAGCAC
CGCTACGATGCAGAGTATTACATCGAGAACCAAGTGCTGCCGGCTGTAGAGCGTATTC
TGAAGGCATACGGTTATCGTAAAGAAGATCAGCGCTATCAAAAGACGAAACAAATTG
GCCTGGGTGCGTGGCTGAAGGTCAAGGGCAAGAAATAA(SEQ ID NO: 12).

According to embodiments of the present disclosure, a nucleotide sequence of the mutant E8 is as follows:

ATGGCGAGCGCGATTCTGGACACTGATTACATTACCGAAAACGGTAAACCGGTTA
TCCGCGTGTTCAAGAAAGAGAATGGTGAGTTCAAAATCGAGTACGATCGCACGTATG
AACCGTACTTCTATGCTCAGCTGAAAGACGTTTCTGCGATTGAAGATGTGAAAAAAGT
GACGGCGAAACGTCACGGCACCGTGGTTAAGGTGAAACGTGCGGAGAAAGTGCAAA
AGAAATTCCTGGGCCGTCCGATCGAAGTTTGGAAGCTGTACTTTAACCACCCACAAG
ACGTCCCGGCGATTCATGACCGCATCCGTGCGCACCCGGCTGTGGTTGACATCTATGA
GTACGATATTCCGTTCGCTAAGAGATACTTGATTGACAAGGGTCTGATCCCTATGGAAG
GTGACGAAGAACTGAAGATGCTGGCGTTCGACATCGAAACTCTGCACCACGAGGGTG
AAGCGTTTGCCGAGGGTCCGATCTTGATGATTTCCTACGCGGACGAAGAGGGCGCAC
GTGTTATCACGTGGAAAAATGTTGATCTGCCGTATGTTGACGTCGTAAGCACCGAGCG
TGAGATGATCAAACGTTTTCTGCGCGTTGTTAAAGAAAAAGATCCTGACGTGCTGATC
ACCTACAACGGTGACAATTTCGATTTCGCGTATCTGAAGAAACGTTGCGAAAAACTGG
GTATTAACTTCGCGCTGGGTCGCGATGGCTCTGAACCGAATATCCTGCGCATGGGTGAT
CGTTTTGCGGTCGAGGTGAAGGGTCGCATTCATTTCGACCTGTACCCGGTGATTCGTC
GTACCACCAACTTGCCGACTTACACCCTGGAAGCCGTCTATGAAGCTGTATTTGGTCA
ACCGAAAGAAAAAGTGTACGCTGTGGAAATTACGACGGCGTGGGAAACCGGTGAGA
GCCTGGAGCGCGTTGCACGTTATTCTATGGAGGACGCGAAAGTTACCTACGAACTGGG
TAAAGAGTTCCTGCCGATGGAGGCCCAACTGTCCCGTCTGGTGGGCCAAAGCCTGTG
GGACGTCAGCCGTTCGTCCACCGGCAACTTGGTTGAATGGTTCCTGCTGCGTAAGGC
ATACAAGCGTAACGAACTGGCGCCGAATAAGCCGGACGAGAAAGAATTGGCGCGTCG
CCGCCAGAGCTATGAGGGTGGTTATGTCAAAGAACCGGAGCGCGGCTTGTGGGAGAA
CATCGTCTATTTGGATTTTCGTAGCATTGCACCGAGCATCATTATCACGCATAATGTGAG
CCCGGATACGTTGAATCGTGAGGGCTGTAAGGAATACGACGTGGCGCCTCAGGTTGG
CCACCGTTTCTGCAAGGACTTTCCGGGCTTTATACCAAGTCTCTTGGGACATTTGTTAG
AGGAAAGACAAAAGATTAAGACAAAAATGAAGGAAACTCAAGATCCTATAGAAAAA
ATACTCCTTGACTATAGACAAAAAGCGATAAAACTCTTAGCAAATTCTTTCTACGGATA

TTATGGCTATGCGCGTGCGCGCTGGTATTGCAAAGAGTGTGCCGAGAGCGTGACCGCT
TGGGGTCGTGAGTACATTACCATGACGATCAAAGAGATTGAAGAGAAATACGGCTTTA
AGGTTATCTATAGCGACACCGACGGTTTCTTTGCAACTATCCCTGGCGCAGACGCAGA
AACCGTTAAGAAAAAGGCAATGGAGTTTCTGAAGTATATCAACGCGAAGTTGCCAGG
CGCCCTGGAACTGGAGTACGAGGGCTTCTACAAGCGTGGCTTTTTCGTGACGAAGAA
GAAGTACGCGGTCATTGACGAAGAGGGCAAGATTACGACCCGTGGTCTGGAAATTGT
TCGCCGTGACTGGTCCGAGATTGCGAAAGAAACCCAGGCGAGAGTGCTGGAAGCGA
TTCTGAAGCATGGTGATGTCGAGGAAGCCGTGCGTATCGTTAAAGAAGTGACGGAGA
AGTTGAGCAAGTACGAAGTCCCACCGGAGAAACTGGTGATTCATGAGCAGATCACGC
GCGATTTACGTGACTATAAAGCAACCGGTCCGCATGTTGCCGTGGCAAAGCGTCTGGC
TGCGCGTGGCGTTAAGATCCGTCCGGGCACGGTTATTAGCTACATTGTGTTGAAAGGT
AGCGGTCGTATTGGCGACCGCGCCATTCCGGCCGACGAGTTCGATCCGACCAAGCAC
CGCTACGATGCAGAGTATTACATCGAGAACCAAGTGCTGCCGGCTGTAGAGCGTATTC
TGAAGGCATACGGTTATCGTAAAGAAGATCAGCGCTATCAAAAGACGAAACAAATTG
GCCTGGGTGCGTGGCTGAAGGTCAAGGGCAAGAAATAA (SEQ ID NO: 13).

According to embodiments of the present disclosure, a nucleotide sequence of the mutant A4-2 is as follows:

ATGGCGAGCGCGATTCTGGACACTGATTACATTACCGAAAACGGTAAACCGGTTA
TCCGCGTGTTCAAGAAAGAGAATGGTGAGTTCAAAATCGAGTACGATCGCACGTATG
AACCGTACTTCTATGCTCTGCTGAAAGACGTTTCTGCGATTGAAGATGTGAAAAAAGT
GACGGCGAAACGTCACGGCACCGTGGTTAAGGTGAAACGTGCGGAGAAAGTGCAAA
AGAAATTCCTGGGCCGTCCGATCGAAGTTTGGAAGCTGTACTTTAACCACCCACAAG
ACGTCCCGGCGATTCATGACCGCATCCGTGCGCACCCGGCTGTGGTTGACATCTATGA
GTACGATATTCCGTTCGCTAAGAGATACTTGATTGACAAGGGTCTGATCCCTATGGAAG
GTGACGAAGAACTGAAGATGCTGGCGTTCGACATCGAAACTCTGTACCACGAGGGTG
AAGCGTTTGCCGAGGGTCCGATCTTGATGATTTCCTACGCGGACGAAGAGGGCGCAC
GTGTTATCACGTGGAAAAATGTTGATCTGCCGTATGTTGACGTCGTAAGCACCGAGCG
TGAGATGATCAAATGTTTTCTGCGCGTTGTTAAAGAAAAAGATCCTGACGTGCTGATC
ACCTACAACGGTGACAATCACGATTTCGCGAATCTGAAGAAACGTTGCGAAAAACTG
GGTATTAACTTCGCGCTGGGTCGCGATGGCTCTGAACCGAATATCCTGCGCATGGGTG
ATCGTTTTGCGGTCGAGGTGAAGGGTCGCATTCATTTCGACCTGTACCCGGTGATTCG
TCGTACCACCAACTTGCCGACTTACACCCTGGAAGCCGTCTATGAAGCTGTATTTGGT

CAACCGAAAGAAAAAGTGTACGCTGTGGAAATTACGACGGCGTGGGAAACCGGTGA
GAGCCTGGAGCGCGTTGCACGTTATTCTATGGAGGACGCGAAAGTTACCTACGAACTG
GGTAAAGAGTTCCTGCCGATGGAGGCCCAACTGTCCCGTCTGGTGGGCCAAAGCCTG
TGGCACGTCAGCCGTTCGTCCACCGGCAACTTGGTTGAATGGTTCCTGCTGCGTAAGG
CATACAAGCGTAACGAACTGGCGCCGAATAAGCCGGACGAGAAAGAATTGGCGCGTC
GCCGCCAGAGCTATGAGGGTGGTTATGTCAAAGAACCGGAGCGCGGCTTGTGGGAGA
ACATCGTCTATTTGGATTTTCGTAGCATTGCACCGAGCATCATTATCACGCATAATGTGA
GCCCGGATACGTTGAATCGTGAGGGCTGTAAGGAATACGACGTGGCGCCTCAGGTTG
GCCACCGTTTCTGCAAGGACTTTCCGGGCTTTATACCAAGTCTCTTGGGACATTTGTTA
GAGGAAAGACAAAAGATTAAGACAAAAATGAAGGAAACTCAAGATCCTATAGAAAA
AATACTCCTTGACTATAGACAAAAAGCGATAAAACTCTTAGCAAATTCTTTCTACGGAT
ATTATGGCTATGCGCGTGCGCGCTGGTATTGCAAAGAGTGTGCCGAGAGCGTGACCGC
TTGGGGTCGTGAGTACATTACCATGACGATCAAAGAGATTGAAGAGAAATACGGCTTT
AAGGTTATCTATAGCGACACCGACGGTTTCTTTGCAACTATCCCTGGCGCAGACGCAG
AAACCGTTAAGAAAAAGGCAATGGAGTTTCTGAAGTATATCAACGCGAAGTTGCCAG
GCGCCCTGGAACTGGAGTACGAGGGCTTCTACAAGCGTGGCTTTTTCGTGACGAAGA
AGAAGTACGCGGTCATTGACGAAGAGGGCAAGATTACGACCCGTGGTCTGGAAATTG
TTCGCCGTGACTGGTCCGAGATTGCGAAAGAAACCCAGGCGAGAGTGCTGGAAGCG
ATTCTGAAGCATGGTGATGTCGAGGAAGCCGTGCGTATCGTTAAAGAAGTGACGGAG
AAGTTGAGCAAGTACGAAGTCCCACCGGAGAAACTGGTGATTCATGAGCAGATCACG
CGCGATTTACGTGACTATAAAGCAACCGGTCCGCATGTTGCCGTGGCAAAGCGTCTGG
CTGCGCGTGGCGTTAAGATCCGTCCGGGCACGGTTATTAGCTACATTGTGTTGAAAGG
TAGCGGTCGTATTGGCGACCGCGCCATTCCGGCCGACGAGTTCGATCCGACCAAGCAC
CGCTACGATGCAGAGTATTACATCGAGAACCAAGTGCTGCCGGCTGTAGAGCGTATTC
TGAAGGCATACGGTTATCGTAAAGAAGATCAGCGCTATCAAAAGACGAAACAAATTG
GCCTGGGTGCGTGGCTGAAGGTCAAGGGCAAGAAATAA(SEQ ID NO: 14).

According to embodiments of the present disclosure, a nucleotide sequence of the mutant QDC4 is as follows:

ATGGCGAGCGCGATTCTGGACACTGATTACATTACCGAAAACGGTAAACCGGTTA
TCCGCGTGTTCAAGAAAGAGAATGGTGAGTTCAAAATCGAGTACGATCGCACGTATG
AACCGTACTTCTATGCTCTGCTGAAAGACGTTTCTGCGATTGAAGATGTGAAAAAAGT
GACGGCGAAACGTCACGGCACCGTGGTTAAGGTGAAACGTGCGGAGAAAGTGCAAA

AGAAATTCCTGGGCCGTCCGATCGAAGTTTGGAAGCTGTACTTTAACCACCCACTGGA
CGTCCCGGCGATTCATGACCGCATCCGTGCGCACCCGGCTGTGGTTGACATCTATGAG
TACGATATTCCGTTCGCTAAGAGATACTTGATTGACAAGGGTCTGATCCCTATGGAAGG
TGACGAAGAACTGAAGATGCTGGCGTTCGACATCGAAACTCTGTACCACGAGGGTGA
AGCGTTTGCCGAGGGTCCGATCTTGATGATTTCCTACGCGGACGAAGAGGGCGCACG
TGTTATCACGTGGAAAAATGTTGATCTGCCGTATGTTGACGTCGTAAGCACCGAGCGT
GAGATGATCAAACGTTTTCTGCGCGTTGTTAAAGAAAAAGATCCTGACGTGCTGATCA
CCTACAACGGTGACAATTTCGATTTCGCGAATCTGAAGAAACGTTGCGAAAAACTGG
GTATTAACTTCGCGCTGGGTCGCGATGGCTCTGAACCGAATATCCTGCGCATGGGTGAT
CGTTTTGCGGTCGAGGTGAAGGGTCGCATTCATTTCGACCTGTACCCGGTGATTCGTC
GTACCACCAACTTGCCGACTTACACCCTGGAAGCCGTCTATGAAGCTGTATTTGGTCA
ACCGAAAGAAAAAGTGTACGCTGTGGAAATTACGACGGCGTGGGAAACCGGTGAGA
GCCTGGAGCGCGTTGCACGTTATTCTATGGAGGACGCGAAAGTTACCTACGAACTGGG
TAAAGAGTTCCTGCCGATGGAGGCCCAACTGTCCCGTCTGGTGGGCCAAAGCCTGTG
GGACGTCAGCCGTTCGTCCACCGGCAACTTGGTTGAATGGTTCCTGCTGCGTAAGGC
ATACAAGCGTAACGAACTGGCGCCGAATAAGCCGGACGAGAAAGAATTGGCGCGTCG
CCGCCAGAGCTATGAGGGTGGTTATGTCAAAGAACCGGAGCGCGGCTTGTGGGAGAA
CATCGTCTATTTGGATTTTCGTAGCATTGCACCGAGCATCATTATCACGCATAATGTGAG
CCCGGATACGTTGAATCGTGAGGGCTGTAAGGAATACGACGTGGCGCCTCAGGTTGG
CCACCGTTTCTGCAAGGACTTTCCGGGCTTTATACCAAGTCTCTTGGGACATTTGTTAG
AGGAAAGACAAAAGATTAAGACAAAAATGAAGGAAACTCAAGATCCTATAGAAAAA
ATACTCCTTGACTATAGACAAAAAGCGATAAAACTCTTAGCAAATTCTTTCTACGGATA
TTATGGCTATGCGCGTGCGCGCTGGTATTGCAAAGAGTGTGCCGAGAGCGTGACCGCT
TGGGGTCGTGAGTACATTACCATGACGATCAAAGAGATTGAAGAGAAATACGGCTTTA
AGGTTATCTATAGCGACACCGACGGTTTCTTTGCAACTATCCCTGGCGCAGACGCAGA
AACCGTTAAGAAAAAGGCAATGGAGTTTCTGAAGTATATCAACGCGAAGTTGCCAGG
CGCCCTGGAACTGGAGTACGAGGGCTTCTACAAGCGTGGCTTTTTCGTGACGAAGAA
GAAGTACGCGGTCATTGACGAAGAGGGCAAGATTACGACCCGTGGTCTGGAAATTGT
TCGCCGTGACTGGTCCGAGATTGCGAAAGAAACCCAGGCGAGAGTGCTGGAAGCGA
TTCTGAAGCATGGTGATGTCGAGGAAGCCGTGCGTATCGTTAAAGAAGTGACGGAGA
AGTTGAGCAAGTACGAAGTCCCACCGGAGAAACTGGTGATTCATGAGCAGATCACGC
GCGATTTACGTGACTATAAAGCAACCGGTCCGCATGTTGCCGTGGCAAAGCGTCTGGC

TGCGCGTGGCGTTAAGATCCGTCCGGGCACGGTTATTAGCTACATTGTGTTGAAAGGT
AGCGGTCGTATTGGCGACCGCGCCATTCCGGCCGACGAGTTCGATCCGACCAAGCAC
CGCTACGATGCAGAGTATTACATCGAGAACCAAGTGCTGCCGGCTGTAGAGCGTATTC
TGAAGGCATACGGTTATCGTAAAGAAGATCAGCGCTATCAAAAGACGAAACAAATTG
GCCTGGGTGCGTGGCTGAAGGTCAAGGGCAAGAAATAA (SEQ ID NO: 15).

According to embodiments of the present disclosure, a nucleotide sequence of the mutant 1-4 is as follows:

ATGGCGAGCGCGATTCTGGACACTGATTACATTACCGAAAACGGTAAACCGGTTA
TCCGCGTGTTCAAGAAAGAGAATGGTGAGTTCAAAATCGAGTACGATCGCACGTTTG
AACCGTACTTCTATGCTCTGCTGAAAGACGATTCTGCGATTGAAGATGTGAAAAAAGT
GACGGCGAAACGTCACGGCACCGTGGTTAAGGTGAAACGTGCGGAGAAAGTGCAAA
AGAAATTCCTGGGCCGTCCGATCGAAGTTTGGAAGCTGTACTTTAACCACCCACAAG
ACGTCCCGGCGATTCGTGACCGCATCCGTGCGCACCCGGCTGTGGTTGACATCTATGA
GTACGATATTCCGTTCGCTAAGAGATACTTGATTGACAAGGGTCTGATCCCTATGGAAG
GTGACGAAGAACTGAAGATGCTGGCGTTCGACATCGAAACTCTGTACCACGAGGGTG
AAGCGTTTGCCGAGGGTCCGATCTTGATGATTTCCTACGCGGACGAAGAGGGCGCAC
GTGTTATCACGTGGAAAAATGTTGATCTGCCGTATGTTGACGTCGTAAGCACCGAGCG
TGAGATGATCAAACGTTTTCTGCGCGTTGTTAAAGAAAAAGATCCTGACGTGCTGATC
ACCTACAACGGTGACAATTTCGATTTCGCGTACCTGAAGAAACGTTGCGAAAAACTG
GGTATTAACTTCGCGCTGGGTCGCGATGGCTCTGAACCGAAGATCCAGCGCATGGGTG
ATCGTTTTGCGGTCGAGGTGAAGGGTCGCATTCATTTCGACCTGTACCCGGTGATTCG
TCGTACCATCAACTTGCCGACTTACACCCTGGAAGCCGTCTATGAAGCTGTATTTGGTC
AACCGAAAGAAAAAGTGTACGCTGTGGAAATTACGACGGCGTGGGAAACCGGTGAG
AGCCTGGAGCGCGTTGCACGTTATTCTATGGAGGACGCGAAAGTTACCTACGAACTGG
GTAAAGAGTTCCTGCCGATGGAGGCCCAACTGTCCCGTCTGGTGGGCCAAAGCCTGT
GGGACGTCAGCCGTTCGTCCACCGGCAACTTGGTTGAATGGTTCCTGCTGCGTAAGG
CATACAAGCGTAACGAACTGGCGCCGAATAAGCCGGACGAGAAAGAATTGGCGCGTC
GCCGCCAGAGCTATGAGGGTGGTTATGTCAAAGAACCGGAGCGCGGCTTGTGGGAGA
ACATCGTCTATTTGGATTTTCGTAGCATTGCACCGAGCATCATTATCACGCATAATGTGA
GCCCGGATACGTTGAATCGTGAGGGCTGTAAGGAATACGACGTGGCGCCTCAGGTTG
GCCACCGTTTCTGCAAGGACTTTCCGGGCTTTATACCAAGTCTCTTGGGACATTTGTTA
GAGGAAAGACAAAAGATTAAGACAAAAATGAAGGAAACTCAAGATCCTATAGAAAA

AATACTCCTTGACTATAGACAAAAAGCGATAAAACTCTTAGCAAATTCTTTCTACGGAT
ATTATGGCTATGCGCGTGCGCGCTGGTATTGCAAAGAGTGTGCCGAGAGCGTGACCGC
TTGGGGTCGTGAGTACATTACCATGACGATCAAAGAGATTGAAGAGAAATACGGCTTT
AAGGTTATCTATAGCGACACCGACGGTTTCTTTGCAACTATCCCTGGCGCAGACGCAG
AAACCGTTAAGAAAAAGGCAATGGAGTTTCTGAAGTATATCAACGCGAAGTTGCCAG
GCGCCCTGGAACTGGAGTACGAGGGCTTCTACAAGCGTGGCTTTTTCGTGACGAAGA
AGAAGTACGCGGTCATTGACGAAGAGGGCAAGATTACGACCCGTGGTCTGGAAATTG
TTCGCCGTGACTGGTCCGAGATTGCGAAAGAAACCCAGGCGAGAGTGCTGGAAGCG
ATTCTGAAGCATGGTGATGTCGAGGAAGCCGTGCGTATCGTTAAAGAAGTGACGGAG
AAGTTGAGCAAGTACGAAGTCCCACCGGAGAAACTGGTGATTCATGAGCAGATCACG
CGCGATTTACGTGACTATAAAGCAACCGGTCCGCATGTTGCCGTGGCAAAGCGTCTGG
CTGCGCGTGGCGTTAAGATCCGTCCGGGCACGGTTATTAGCTACATTGTGTTGAAAGG
TAGCGGTCGTATTGGCGACCGCGCCATTCCGGCCGACGAGTTCGATCCGACCAAGCAC
CGCTACGATGCAGAGTATTACATCGAGAACCAAGTGCTGCCGGCTGTAGAGCGTATTC
TGAAGGCATACGGTTATCGTAAAGAAGATCAGCGCTATCAAAAGACGAAACAAGTTG
GCCTGGGTGCGTGGCTGAAGGTCAAGGGCAAGAAATAA (SEQ ID NO: 16).

According to embodiments of the present disclosure, a nucleotide sequence of the mutant QAA1 is as follows:

ATGGCGAGCGCGATTCTGGACACTGATTACATTACCGAAAACGGTAAACCGGTTA
TCCGCGTGTTCAAGAAAGAGAATGGTGAGTTCAAAATCGAGTACGATCGCACGTATG
AACCGTACTTCTATGCTCTGCTGAAAGACGTTTCTGCGATTGAAGATGTGAAAAAAGT
GACGGCGAAACGTCACGGCACCGTGGTTAAGGTGAAACGTGCGGAGAAAGTGCAAA
AGAAATTCCTGGGCCGTCCGATCGAAGTTTGGAAGCTGTACTTTAACCACCCACACGA
CGTCCCGGCGATTCATGACCGCATCCGTGCGCACCCGGCTGTGGTTGACATCTATGAG
TACGATATTCCGTTCGCTAAGAGATACTTGATTGACAAGGGTCTGATCCCTATGGAAGG
TGACGAAGAACTGAAGATGCTGGCGTTCGACATCGAAACTCTGTACCACGAGGGTGA
AGCGTTTGCCGAGGGTCCGATCTTGATGATTTCCTACGCGGACGAAGAGGGCGCACG
TGTTATCACGTGGAAAAATGTTGATCTGCCGTATGTTGACGTCGTAAGCACCGAGCGT
GAGATGATCAAACGTTTTCTGCGCGTTGTTAAAGAAAAAGATCCTGACGTGCTGATCA
CCTACAACGGTGACAATTTCGATTTCGCGAATCTGAAGAAACGTTGCGAAAAACTGG
GTATTAACTTCGCGCTGGGTCGCGATGGCTCTGAACCGAATATCCTGCGCATGGGTGAT
CGTTTTGCGGTCGAGGTGAAGGGTCGCATTCATTTCGACCTGTACCCGGTGATTCGTC

GTACCACCAACTTGCCGACTTACACCCTGGAAGCCGTCTATGAAGCTGTATTTGGTCA
ACCGAAAGAAAAAGTGTACGCTGTGGAAATTACGACGGCGTGGGAAACCGGTGAGA
GCCTGGAGCGCGTTGCACGTTATTCTATGGAGGACGCGAAAGTTACCTACGAACTGGG
TAAAGAGTTCCTGCCGATGGAGGCCCAACTGTCCCGTCTGGTGGGCCAAAGCCTGTG
GGACGTCAGCCGTTCGTCCACCGGCAACTTGGTTGAATGGTTCCTGCTGCGTAAGGC
ATACAAGCGTAACGAACTGGCGCCGAATAAGCCGGACGAGAAAGAATTGGCGCGTCG
CCGCCAGAGCTATGAGGGTGGTTATGTCAAAGAACCGGAGCGCGGCTTGTGGGAGAA
CATCGTCTATTTGGATTTTCGTAGCATTGCACCGAGCATCATTATCACGCATAATGTGAG
CCCGGATACGTTGAATCGTGAGGGCTGTAAGGAATACGACGTGGCGCCTCAGGTTGG
CCACCGTTTCTGCAAGGACTTTCCGGGCTTTATACCAAGTCTCTTGGGACATTTGTTAG
AGGAAAGACAAAAGATTAAGACAAAAATGAAGGAAACTCAAGATCCTATAGAAAAA
ATACTCCTTGACTATAGACAAAAAGCGATAAAACTCTTAGCAAATTCTTTCTACGGATA
TTATGGCTATGCGCGTGCGCGCTGGTATTGCAAAGAGTGTGCCGAGAGCGTGACCGCT
TGGGGTCGTGAGTACATTACCATGACGATCAAAGAGATTGAAGAGAAATACGGCTTTA
AGGTTATCTATAGCGACACCGACGGTTTCTTTGCAACTATCCCTGGCGCAGACGCAGA
AACCGTTAAGAAAAAGGCAATGGAGTTTCTGAAGTATATCAACGCGAAGTTGCCAGG
CGCCCTGGAACTGGAGTACGAGGGCTTCTACAAGCGTGGCTTTTTCGTGACGAAGAA
GAAGTACGCGGTCATTGACGAAGAGGGCAAGATTACGACCCGTGGTCTGGAAATTGT
TCGCCGTGACTGGTCCGAGATTGCGAAAGAAACCCAGGCGAGAGTGCTGGAAGCGA
TTCTGAAGCATGGTGATGTCGAGGAAGCCGTGCGTATCGTTAAAGAAGTGACGGAGA
AGTTGAGCAAGTACGAAGTCCCACCGGAGAAACTGGTGATTCATGAGCAGATCACGC
GCGATTTACGTGACTATAAAGCAACCGGTCCGCATGTTGCCGTGGCAAAGCGTCTGGC
TGCGCGTGGCGTTAAGATCCGTCCGGGCACGGTTATTAGCTACATTGTGTTGAAAGGT
AGCGGTCGTATTGGCGAGCGCGCCATTCCGGCCGACGAGTTCGATCCGACCAAGCAA
CGCTACGATGCAGAGTATTACATCGAGAACCAAGTGCTGCCGGCTGTAGAGCGTATTC
TGAAGGCATACGGTTATCGTAAAGAAGATCAGCGCTATCAAAAGACGAAACAAATTG
GCCTGGGTGCGTGGCTGAAGGTCAAGGGCAAGAAATAA (SEQ ID NO: 17)

According to embodiments of the present disclosure, a nucleotide sequence of the mutant QAA3 is as follows:

ATGGCGAGCGCGATTCTGGACACTGATTACATTACCGAAAACGGTAAACCGGTTA
TCCGCGTGTTCAAGAAAGAGAATGGTGAGTTCAAAATCGAGTACGATCGCACGTATG
AACCGTACTTCTATGCTCTGCTGAAAGACGTTTCTGCGATTGAAGATGTGAAAAAAGT

GACGGCGAAACGTCACGGCACCGTGGTTAAGGTGAAACGTGCGGAGAAAGTGCAAA
AGAAATTCCTGGGCCGTCCGATCGAAGTTTGGAAGCTGTACTTTAACCACCCACTGGA
CGTCCCGGCGATTCATGACCGCATCCGTGCGCACCCGGCTGTGGTTGACATCTATGAG
TACGATATTCCGTTCGCTAAGAGATACTTGATTGACAAGGGTCTGATCCCTATGGAAGG
TGACGAAGAACTGAAGATGCTGGCGTTCGACATCGAAACTCTGTACCACGAGGGTGA
AGCGGCTGCCGAGGGTCCGATCTTGATGATTTCCTACGCGGACGAAGAGGGCGCACG
TGTTATCACGTGGAAAAATGTTGATCTGCCGTATGTTGACGTCGTAAGCACCGAGCGT
GAGATGATCAAACGTTTTCTGCGCGTTGTTAAAGAAAAAGATCCTGACGTGCTGATCA
CCTACAACGGTGACAATTTCGATTTCGCGAATCTGAAGAAACGTTGCGAAAAACTGG
GTATTAACTTCGCGCTGGGTCGCGATGGCTCTGAACCGAATATCCTGCGCATGGGTGAT
CGTTTTGCGGTCGAGGTGAAGGGTCGCATTCATTTCGACCTGTACCCGGTGATTCGTC
GTACCACCAACTTGCCGACTTACACCCTGGAAGCCGTCTATGAAGCTGTATTTGGTCA
ACCGAAAGAAAAAGTGTACGCTGTGGAAATTACGACGGCGTGGGAAACCGGTGAGA
GCCTGGAGCGCGTTGCACGTTATTCTATGGAGGACGCGAAAGTTACCTACGAACTGGG
TAAAGAGTTCCTGCCGATGGAGGCCCAACTGTCCCGTCTGGTGGGCCAAAGCCTGTG
GGACGTCAGCCGTTCGTCCACCGGCAACTTGGTTGAATGGTTCCTGCTGCGTAAGGC
ATACAAGCGTAACGAACTGGCGCCGAATAAGCCGGACGAGAAAGAATTGGCGCGTCG
CCGCCAGAGCTATGAGGGTGGTTATGTCAAAGAACCGGAGCGCGGCTTGTGGGAGAA
CATCGTCTATTTGGATTTTCGTAGCATTGCACCGAGCATCATTATCACGCATAATGTGAG
CCCGGATACGTTGAATCGTGAGGGCTGTAAGGAATACGACGTGGCGCCTCAGGTTGG
CCACCGTTTCTGCAAGGACTTTCCGGGCTTTATACCAAGTCTCTTGGGACATTTGTTAG
AGGAAAGACAAAAGATTAAGACAAAAATGAAGGAAACTCAAGATCCTATAGAAAAA
ATACTCCTTGACTATAGACAAAAAGCGATAAAACTCTTAGCAAATTCTTTCTACGGATA
TTATGGCTATGCGCGTGCGCGCTGGTATTGCAAAGAGTGTGCCGAGAGCGTGACCGCT
TGGGGTCGTGAGTACATTACCATGACGATCAAAGAGATTGAAGAGAAATACGGCTTTA
AGGTTATCTATAGCGACACCGACGGTTTCTTTGCAACTATCCCTGGCGCAGACGCAGA
AACCGTTAAGAAAAAGGCAATGGAGTTTCTGAAGTATATCAACGCGAAGTTGCCAGG
CGCCCTGGAACTGGAGTACGAGGGCTTCTACAAGCGTGGCTTTTTCGTGACGAAGAA
GAAGTACGCGGTCATTGACGAAGAGGGCAAGATTACGACCCGTGGTCTGGAAATTGT
TCGCCGTGACTGGTCCGAGATTGCGAAAGAAACCCAGGCGAGAGTGCTGGAAGCGA
TTCTGAAGCATGGTGATGTCGAGGAAGCCGTGCGTATCGTTAAAGAAGTGACGGAGA
AGTTGAGCAAGTACGAAGTCCCACCGGAGAAACTGGTGATTCATGAGCAGATCACGC

GCGATTTACGTGACTATAAAGCAACCGGTCCGCATGTTGCCGTGGCAAAGCGTCTGGC
TGCGCGTGGCGTTAAGATCCGTCCGGGCACGGTTATTAGCTACATTGTGTTGAAAGGT
AGCGGTCGTATTGGCGACCGCGCCATTCCGGCCGACGAGTTCGATCCGACCAAGCAC
CGCTACGATGCAGAGTATTACATCGAGAACCAAGTGCTGCCGGCTGTAGAGCGTATTC
TGAAGGCATACGGTTATCGTAAAGAAGATCAGCGCTATCAAAAGACGAAACAAATTG
GCCTGGGTGCGTGGCTGAAGGTCAAGGGCAAGAAATAA (SEQ ID NO: 18).

### Construct

In still another aspect, the present disclosure provides in embodiments a construct. According to embodiments of the present disclosure, the construct contains the isolated nucleic acid as described above. The construct according to embodiments of the present disclosure can be used to express the chimeric DNA polymerase having the properties of high yield for amplifying products, high specificity, high continuous synthesis ability, high extension rate, thermal stability, strong resistance to salt, high fidelity, etc., therefore meeting the needs of DNA amplification, synthesis, detection, sequencing, etc., and having a broad application prospect.

It will be appreciated by those skilled in the art that the features and advantages described above for the isolated nucleic acid are also applicable to the construct, and thus will not be repeated herewith.

### Recombinant cell or recombinant microorganism

In yet another aspect, the present disclosure provides in embodiments a recombinant cell or a recombinant microorganism. According to embodiments of the present disclosure, the recombinant cell or recombinant microorganism includes the isolated nucleic acid as described above. Accordingly, the recombinant cell or a recombinant microorganism according to embodiments of the present disclosure can express the chimeric DNA polymerase having the properties of high yield for amplifying products, high specificity, high continuous synthesis ability, high extension rate, thermal stability, strong resistance to salt, high fidelity, etc., therefore meeting the needs of DNA amplification, synthesis, detection, sequencing, etc., and having a broad application prospect.

It should be noted that the recombinant cell in embodiments of the present disclosure does not include germ cells, fertilized eggs, embryonic cells and etc. of animals, and does not belong to animal species.

It will be appreciated by those skilled in the art that the features and advantages described above for the isolated nucleic acid are also applicable to the recombinant cell or the recombinant microorganism, and thus will not be repeated herewith.

### Method for obtaining chimeric DNA polymerase

In yet another aspect, the present disclosure provides in embodiments a method for obtaining the chimeric DNA polymerase. According to embodiments of the present disclosure, the method includes: cultivating the recombinant cell or the recombinant microorganism described above in a condition suitable for expressing the chimeric DNA polymerase, so as to obtain the chimeric DNA polymerase. Accordingly, with the method according to embodiments of the present disclosure, the chimeric DNA polymerase having the properties of high yield for amplifying products, high specificity, high continuous synthesis ability, high extension rate, thermal stability, strong resistance to salt, high fidelity, etc. can be obtained, therefore meeting the needs of DNA amplification, synthesis, detection, sequencing, etc., and having a broad application prospect.

It will be appreciated by those skilled in the art that the features and advantages described above for the recombinant cell or the recombinant microorganism are also applicable to the method, and thus will not be repeated herewith.

### Kit

In yet another aspect, the present disclosure provides in embodiments a kit. According to embodiments of the present disclosure, the kit includes the chimeric DNA polymerase, the isolated nucleic acid, the construct, or the recombinant cell or the recombinant microorganism as described above. Therefore, DNA amplification by using the kit according to embodiments of the present disclosure has the advantages of high yield of amplification products, high amplification accuracy and so on, and is suitable for widespread production and application.

It will be appreciated by those skilled in the art that the features and advantages described above for the chimeric DNA polymerase, the isolated nucleic acid, the construct, the recombinant cell or the recombinant microorganism are also applicable to the kit, and thus will not be repeated herewith.

### Use

In yet another aspect, the present disclosure provides in embodiments use of the chimeric DNA polymerase, the isolated nucleic acid, the construct, the recombinant cell or recombinant microorganism, or the kit described above for DNA amplification. Therefore, such DNA amplification has the advantages of high yield of amplification products, high amplification accuracy and so on, and is suitable for widespread production and application.

According to embodiments of the present disclosure, the chimeric DNA polymerase, the isolated nucleic acid, the construct, the recombinant cell or the recombinant microorganism, or the kit is used for gene screening, sequencing or mutation detection.

It will be appreciated by those skilled in the art that the features and advantages described above for the chimeric DNA polymerase, the isolated nucleic acid, the construct, the recombinant cell or the recombinant microorganism, and the kit are also applicable to the use, and thus will not be repeated herewith.

Embodiments of the disclosure will be described in detail below in connection with the Examples, but it will be appreciated by those skilled in the art that the following Examples are only intended to illustrate the present disclosure and should not be regarded as limiting the scope of the present disclosure. Where specific techniques or conditions are not indicated in the Examples, they are performed in accordance with the techniques or conditions described in the literature in the art or in accordance with the product specification. The reagents or instruments used, where no manufacturer is indicated, are conventional products available through the market.

### Example 1: Design and construction of chimeric DNA polymerase

Pfu, 9⁰N and KOD DNA polymerases are all derived from archaeobacteria. They have good thermo-resistance and proofreading performance, but different phenotypic characteristics. Among all DNA polymerases with thermal stability and fidelity, Pfu DNA polymerase has the lowest error probability for amplification with an error rate of about 2.0×10⁻⁶; 9⁰N DNA polymerase, with the same fidelity, has a higher affinity with double stranded DNA than Pfu DNA polymerase; and KOD DNA polymerase has high amplification ability with amplification yield of ~300nts, and an amplification speed twice as that of Taq DNA polymerase and six times as that of Pfu DNA polymerase.

The novel chimeric DNA polymerase in this example is a chimeric combination of Pfu, 9⁰N and KOD DNA polymerases (as shown in Figure 1), which shows high thermal stability, salt tolerance and exonuclease activity. Specifically, a. nucleotide sequences at (i) positions 1-390 and 1015-1116, and (ii) positions 1771-2328, of the nucleotide sequence for 9⁰ N DNA polymerase, drawn to (i) a N-terminal domain and (ii) a thumb domain of 9⁰ N DNA polymerase, respectively; b. nucleotide sequences at (i) positions 391-1014, and (ii) positions 1117-1341 and 1498-1770, of the nucleotide sequence for KOD DNA polymerase, drawn to (i) an exonucleolytic domain and (ii) palm domain of KOD DNA polymerase, respectively; and c. a nucleotide sequence at positions 1345-1500 of the nucleotide sequence for Pfu DNA polymerase, drawn to a finger domain of Pfu DNA polymerase, were introduced into a prokaryotic expression vector pET28a between its *XhoI*/*BamHI* restriction sites, and transformed into *E*. *coli* BL21 (DE3). After culture, an expressing strain was obtained.

### Example 2: Fermentation expression and purification of the chimeric DNA polymerase and mutants thereof

### 2.1 Fermentation expression

The obtained expressing strain was inoculated, at a scale of 1:100, into a liquid LB medium containing kanamycin, and was incubated at 37 °C with 220 rpm until OD600=0.6. Then 0.5 mM IPTG was added and the strain was induced for expression overnight at low temperature (16 °C) with 220 rpm (for 16 h). After that, the induced strain was centrifuged at 6000 rpm for 8 min to collect bacterial precipitation.

### 2.2 Treatment for fermented bacteria

The bacteria were resuspended with a bacteria suspension solution A at a ratio of the bacteria weight (g) to the bacteria suspension solution A (ml) (20 mM Tris, 300 mM NaCl, 20 mM Imidazole, 5% Glycerol, pH7.4) = 1:20, and were subject to ultrasonication. Then, the solution was centrifuged at 12000 rpm for 20 min to collect the supernatant after sonication. The supernatant was denatured in a water bath at 75 °C for 30 min, and then centrifuged at 12000 rpm for 20 min to recover the supernatant.

### 2.3 Purification with Ni column

The recovered supernatant was filtered through 0.22 µm filtration device and then the filtered solution was injected into a Ni column, which had been washed and balanced with the bacterial suspension solution A. The concentration of imidazole in an eluent (20 mM Tris, 300 mM NaCl, 5% Glycerol, 500 mM Imidazole, pH7.4) was adjusted for gradient elution. The fraction from the column was collected and the active fraction in which was analyzed through SDS-PAGE. The fractions of pure target proteins observed on SDS-PAGE gel stained by Coomassie were merged.

### 2.4 Purification with anion column

The merged fractions above were passed through an anion column so as to control the residual endonuclease and nucleic acid in the sample. The merged fractions were dialyzed into Buffer C (20 mM Tris, 50 mM NaCl, 5% Glycerol, pH7.4), and subject to gradient elution by adjusting the concentration of salt ions in Buffer D (20 mM Tris, 500 mM NaCl, 5% Glycerol, pH7.4), and the fraction collected from the elution column was the novel chimeric DNA polymerase.

### 2.5 Purification with cation column

The collected sample after anion column purification was further passed through a cation column to increase the concentration. The collected sample from the anion column was dialyzed into Buffer C (20 mM Tris, 50 mM NaCl, 5% Glycerol, pH7.4), and subject to gradient elution by adjusting the concentration of salt ions in Buffer D (20 mM Tris, 500 mM NaCl, 5% Glycerol, pH7.4). The collected fractions from the elution column were the novel chimeric DNA polymerase. The obtained sample was dialyzed to a preservation system (20 mM Tris, 100 mM KCl, 50% Glycerol, 0.1 mM EDTA, 1 mM DTT, 0.001% Tween20, 0.001% NP40, pH7.4).

### Example 3: Amplification performance and salt tolerance of the novel chimeric DNA polymerase

Using *E.coli* gDNA as a template, the novel chimeric DNA polymerase obtained in Examples 1 and 2 of the present disclosure was subjected to amplification, with an amplified fragment of 1.5 kb.

Primers used were as follows:
Ecoli-F: AGAGTTTGATCMTGGCTCAG (SEQ ID NO: 25);
Ecoli-R: CGGTTACCTTGTTACGACTT (SEQ ID NO: 26).

The reaction procedure and system of the amplification are as follows. The amplification results are shown in FIG. 3.

**Table 1: Salt tolerance assay on the novel chimeric DNA polymerase**

| Temperature | Time | The number of cycles | | Components | Volume (µl) |
|---|---|---|---|---|---|
| 95 °C | 3 min | 1 | | 5x PCR Buffer | 5 |
| 98 °C | 20 sec | 30 | | *E.coli* gDNA (10 ng/µl) | 1 |
| 61 °C | 15 sec | | | Primer (10 µM) | 1 for each |
| 72 °C | 70 sec | | | dNTPs (10 mM) | 1.75 |
| 72 °C | 5 min | 1 | | KCl | 10-160 mM |
| | | | | polymerase | 0.5 |
| 8 °C | ∞ | 1 | | H2O | Made up to 25 µl |

The reaction products were detected by agarose gel electrophoresis, and the results are shown in FIG. 3. The results showed that when KCl was added to 80 mM, the novel chimeric DNA polymerase still could perform amplification well. Compared with KOD and Pfu DNA polymerases which were widely used at present, the amplification yield of the novel chimeric DNA polymerase was not lower than that of KOD DNA polymerase, and the salt tolerance of the novel chimeric DNA polymerase was higher than that of Pfu DNA polymerase.

### Example 4: Assay on thermal stability of the chimeric DNA polymerase

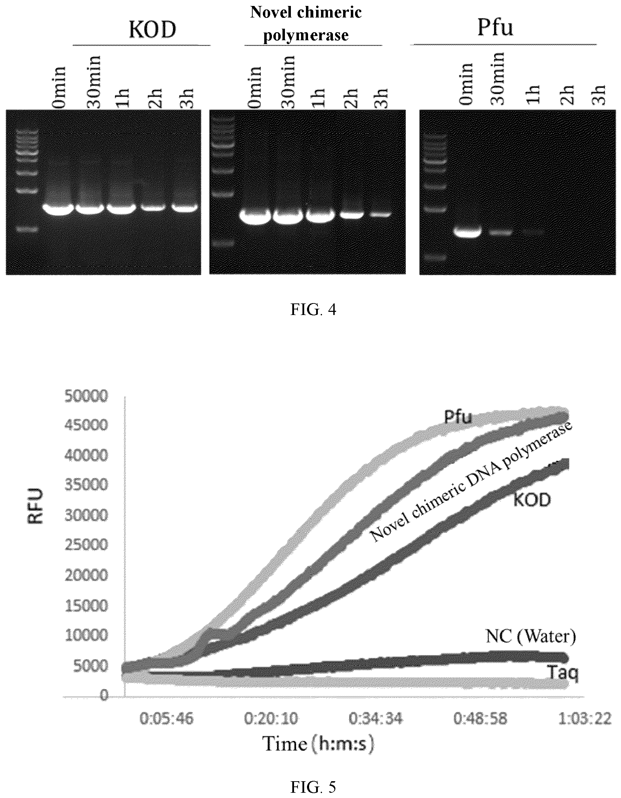

The novel chimeric DNA polymerase was incubated at 98 °C for 0, 30, 60, 120 or 180 minutes. After that, the incubated polymerase was used to amplify *E. coli* gDNA, and PCR products of the amplification were analyzed through agarose gel. The amplification system and procedure were referred to Example 3. The results are shown in FIG. 4.

The results showed that the thermal resistance of the novel chimeric DNA polymerase was better than that of Pfu and KOD DNA polymerases, which were widely used at present. At all time points during the assay, the thermal resistance of the novel chimeric DNA polymerase was at the same level as that of KOD DNA polymerase.

### Example 5: Assay on 3'- 5' exonucleolytic activity of the chimeric DNA polymerase

The assay on exonucleolytic activity adopted double stranded mismatch substrate method with fluorescence probe. There were three non-complementary bases failing to pairing at respective ends of strand A and strand B, in which quenching group BHQ2 was linked at the 3' end of strand A, and quenching fluorophore Rox was linked at the 5' end of strand B. The 3'- 5' exonucleolytic activity of the chimeric DNA polymerase rendered cleavage to the mismatch bases in the A-B double strands, and the generated fluorescence was detected by a microplate reader. The reaction system and conditions for exonucleolytic activity assay are shown in Table 2.

**Table2: Assay on exonucleolytic activity of the novel chimeric DNA polymerase**

| Reagent | Volume |
|---|---|
| 5x PCR buffer | 5 µL |
| A-B double stranded substrate | 0.5 µL |
| 25 mM dNTP | 1 µL |
| polymerase | 1 µL |
| ddH₂O | Made up to the final volume of 50 µL |
| 37 °C, for 1 h, with fluorescence detection every 8 s, 582/618 nm | |

The results (FIG. 5) showed that the novel chimeric DNA polymerase had significant 3'- 5' exonuclease activity, which was higher than that of KOD DNA polymerase.

### Example 6: Directed evolution experiment based on the chimeric DNA polymerase

Directed evolution experiments were designed to obtain mutant polymerases that are more suitable for recombinant DNA technology. By simply imitating normal PCR conditions at which the polymerases are commonly used, or undesirable PCR conditions, a polymerase (or multiple polymerases) that was more suitable for the typical application of recombinant DNA technology should appear after sufficient rounds of selection.

The specific steps are as follows: on the basis of the novel chimeric DNA polymerase as constructed, a mutant library of chimeric DNA polymerases was generated by error prone PCR. Expression vectors for the corresponding mutant library were constructed and expressed with fermentation, and the mutant polymerases were subject to amplification under specific PCR conditions, for example, shortened extension time, reduced amplification cycles, harsh PCR components, such as high salt, etc., to obtain mutants with improved amplification performance, as such this round of mutant evolution screening was completed.

Further, based on the positive transformants obtained from the previous round of screening, the next round of mutant library was generated through error prone PCR, and the mutants with improved target performance were screened out according to specific performance such as amplification yield, long fragment amplification ability, amplification ability for low template input, amplification specificity and fidelity, etc. In a similar fashion, final mutants were obtained through seven rounds of directed evolution of polymerase.

The amplification system for mutant library construction by error prone PCR is shown in Table 3. The corresponding amplification procedure is shown in Table 4.

**Table 3 Mutant library construction**

| Components | Volume |
|---|---|
| 10*PCR buffer | 5 µl |
| dNTP (10 mM) | 1 µl |
| dCTP (40 mM) | 1 µl |
| dTTP (40 mM) | 1 µl |
| MgCl₂ (55 mM) | 0.01-1 mM |
| MnCl₂ (1 mM) | 3-7 mM |
| Primer-F (10 µM) | 0.5 µl |
| Primer-R (10 µM) | 0.5 µl |
| gene template | 20-50 ng |
| Taq DNA polymerase (5 U/µl) | 0.5 µl |
| H₂O | Made up to 50 µl |

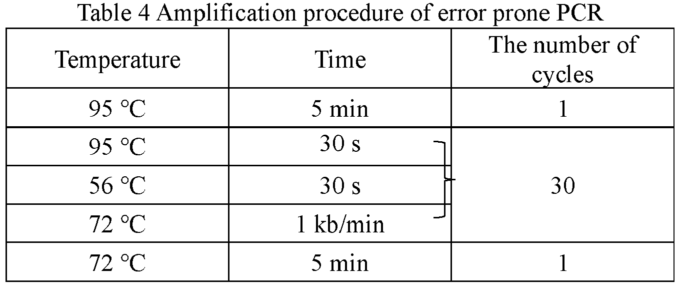

### Example 7: Mutant screening under high salt conditions or shortened extension times

The mutant polymerases obtained through construction, fermentation, and purification in Example 6 was screened according to the resistance of each mutant to high salt (100 mM of KCl) or shortened extension rate (30 s/kb) of PCR amplification in the PCR reaction. The amplification system and amplification procedure are referred to Example 3. The reaction products were detected by agarose gel electrophoresis.

The identified mutations and their corresponding positions are shown in Table 5. Based on the high salt resistance (100mM KCl) and enhanced elongation rate, the identified clones of mutations or mutation combinations are shown in Table 6, as examples.

**Table 5: Mutations identified in chimeric polymerase mutant clones selected for high salt resistance or PCR amplification extension rate**

| Position | Mutation | Position | Mutation | Position | Mutation |
|---|---|---|---|---|---|
| 37 | F37Y | 217 | F217G | 482 | L482Q |
| 44 | L44Q | 217 | F217H | 520 | G520A |
| 48 | D48V | 219 | F219L | 528 | I528V |
| 77 | K77R | 221 | Y221N | 535 | Y535N |
| 94 | Q94H | 243 | K243N | 540 | I540V |
| 94 | Q94L | 245 | Q245L | 598 | A598T |
| 100 | R100H | 257 | G257A | 614 | V614I |
| 101 | D101K | 271 | I271T | 650 | T650A |
| 137 | E137K | 296 | E296V | 667 | E667V |
| 149 | Y149H | 304 | T304I | 715 | D715E |
| 154 | E154A | 307 | N307S | 719 | P719S |
| 155 | F155A | 332 | M332T | 728 | H728Q |
| 155 | F155K | 346 | D346H | 745 | E745K |
| 157 | E157D | 377 | E377K | 751 | F751Y |
| 162 | M162I | 382 | R382G | 758 | L758Q |
| 176 | W176R | 394 | E394H | 766 | V766I |
| 196 | R196C | 434 | Y434N | 777 | K777R |

**Table 6: Clones, as examples, of identified mutations or mutations combinations selected for high salt resistance (KCl) or enhanced extension rate**

| Clone name | Mutation |
|---|---|
| 1-3 | M162I, 1540V, A598T, H728Q |
| 1-4 | E296V, N307S, F751Y, L758Q, E154A |
| 2-3 | G257A, E296V, N307S, M332T, Y434N, L482Q, Y535N, V614I, F751Y, L758Q, E514A |
| E5 | F37Y, D48V, R100H, Y221N, K243N, Q245L, I271T, E296V, N307S, F751Y, L758Q, V766I, E154A |
| E8 | F37Y, L44Q, D48V, R100H, Y149H, K243N, Q245L, I271T, E296V, N307S, F751Y, L758Q, V766I, E154A |
| B4 | F37Y, L44Q, D48V, Q94L, R100H, K243N, Y149H, W176R, Q245L, I271T, E296V, N307S, 1528V, E667V, F751Y, L758Q, V766I, E154A |
| QAA1 | F37Y, D48V, Q94H,R100H, Y221N, K243N, Q245L, I271T, E296V, N307S, D715E, H728Q, F751Y, L758Q, V766I, E154A |
| QAA3 | F37Y, D48V, Q94L, R100H, F155A, Y221N, K243N, Q245L, I271T, E296V, N307S, F751Y, L758Q, V766I, E154A |
| 2D5 | F37Y, D48V, R100H, Y221N, K243N, Q245L, I271T, E296V, N307S, F751Y, L758Q, V766I, E154A, Q94L, M162I, I528V, E667V, H728Q |
| 1C5 | F37Y, D48V, R100H, Y221N, K243N, Q245L, I271T, E296V, N307S, F751Y, L758Q, V766I, E154A, K77R, Q94L, M162I, 1540V, H728Q |
| 346H-1 | F37Y, D48V, R100H, R196C, Y221N, K243N, Q245L, I271T, E296V, N307S, D346H, F751Y, L758Q, V766I, E154A |
| A3-2 | F37Y, D48V, Q94L, R100H, Y149H, Y221N, K243N, Q245L, I271T, E296V, N307S, R382G, F751Y, L758Q, V766I, P719S, E154A |
| 2C6 | F37Y, D48V, Q94L, R100H, Y221N, K243N, Q245L, I271T, E296V, N307S, I528V, 1540V, A598T, E667V, H728Q, F751Y, L758Q, V766I, E154A |
| K5D2 | F37Y, D48V, Q94H, R100H, D101K, Y221N, K243N, Q245L, I271T, E296V, N307S, E377K, E745K, F751Y, L758Q, V766I, K777R, E154A |
| 155A-6 | F37Y, D48V, R100H, F155A, Y221N, K243N, Q245L, I271T, E296V, N307S, F751Y, L758Q, V766I, E154A |
| 1D4 | F37Y, D48V, Q94L, R100H, M162I, Y221N, K243N, Q245L, I271T, E296V, N307S, 1528V, 1540V, H728Q, F751Y, L758Q, V766I, E154A |
| 394H-5 | F37Y, D48V, R100H, Y221N, K243N, Q245L, I271T, E296V,T304I, N307S, E394H, F219L, F751Y, L758Q, V766I, E154A |
| KAC4 | F37Y, D48V, R100H, Y221N, K243N, Q245L, I271T, E296V, N307S, F751Y, L758Q, V766I, E154A, G520A |
| K4D5 | F37Y, D48V, R100H, M162I, W176R, Y221N, K243N, Q245L, I271T, E296V, N307S, I540V, E667V, H728Q, F751Y, L758Q, V766I, E154A |
| K4B6 | F37Y, D48V, R100H, M162I, W176R, Y221N, K243N, Q245L, I271T, E296V, N307S, 1540V, E667V, H728Q, F751Y, L758Q, V766I, K777R, E154A |
| 1D6 | F37Y, D48V, Q94L, R100H, M162I, W176R, Y221N, K243N, Q245L, I271T, E296V, N307S, 1540V, E667V, H728Q, F751Y, L758Q, V766I, E154A |
| K5A3 | F37Y, D48V, Q94H, R100H, D101K, F155K, Y221N, K243N, Q245L, I271T, E296V, N307S, E745K, F751Y, L758Q, V766I, E154A |
| A4-2 | F37Y, D48V, R100H, R196C, F217H,Y221N, K243N, Q245L, I271T, E296V, N307S, D346H, F751Y, L758Q, V766I, E154A |
| QDC4 | F37Y, D48V, Q94L, R100H,Y221N, K243N,Q245L, I271T, E296V, N307S, F751Y, L758Q, V766I, E154A |

### Example 8: Screening mutants suitable for amplification with low template input

In order to screen out and obtain mutants suitable for PCR amplification under the condition of low template input, mutants were subject to amplification with 50 µL PCR amplification system, where 100 pg of human genome were input to amplify gene hGABARAPL2, thereby testing the amplification ability of the mutant. The primer sequences used are as follows:
hGABARAPL2-F: CCAGCCAATTCATGAGTCGGTG (SEQ ID NO: 27);
hGABARAPL2-R: CCTGACAACTCGCAAGTAGCAC (SEQ ID NO: 28).

The reaction procedure and system for amplification are shown in Table 7.

**Table 7: Amplification reaction procedure and system for mutant screening under low-template input**

| Temperature | Time | The number of cycles | | Components | Volume (µl) |
|---|---|---|---|---|---|
| 95 °C | 3 min | 1 | | 5x PCR Buffer | 10 |
| 98 °C | 20 sec | 30 | | Human gDNA (100 pg/µl) | 1 |
| 61 °C | 20 sec | | | Primer (10 µM) | 2 for each |
| 72 °C | 20 s | | | dNTPs (10 mM) | 2.5 |
| 72 °C | 5 min | 1 | | Polymerase | 1 |
| 8 °C | ∞ | 1 | | H₂O | Made up to 50 µl |

The reaction products were detected by agarose gel electrophoresis. Clones of mutant chimeric polymerases, based on wild type chimeric DNA polymerase and identified in amplification under low template input are shown in Table 8, as examples.

**Table 8: Mutant clones of chimeric polymerases screened out suitable for low template input**

| Clone name |
|---|
| 1-3 |
| E5 |
| E8 |
| QAA1 |
| QAA3 |
| 346H-1 |
| A3-2 |
| 155A-6 |
| KAC4 |
| K5A3 |
| A4-2 |
| QDC4 |

### Example 9: Screening for mutant suitable for long fragment amplification

In order to screen out and obtain mutants suitable for long fragment amplification, primer pairs were used to generate 6 kb, 8 kb, or 10 kb of fragments based on lambda DNA templates. Under a limited polymerase concentration, each mutant was tested for the ability to continuously synthesize fragment of each length. The primer sequences used are as follows:
lam-F: CCTCTGTCGTTTCCTTTCTCTGTTTTTGTCCGTGG (SEQ ID NO: 29);
lam6K-R: ACATCGACATAAAAAAATCCCGTAAAAAAAGCCGCA (SEQ ID NO: 30);
lam8K-R: CGGGAATACGACGGTTACCCACCACAAGCACG (SEQ ID NO: 31);
lam10K-R: GCCGCATCCAGACTCAAATCAACGACCAGA (SEQ ID NO: 32).

Refer to Example 8 for amplification reaction procedure and system, in which the extension rate was set to 45 s/kb, and the lambda DNA template input for 100 pg. The reaction products were detected by agarose gel electrophoresis. Clones of mutant chimeric polymerases, based on wild type chimeric DNA polymerase and identified in long fragment amplification, are shown in Table 9, as examples.

**Table 9: Chimeric polymerase mutant clones screened out for long fragment amplification**

| Clone name | 6 kb | 8 kb | 10 kb |
|---|---|---|---|
| 1-3 | yes | no | no |
| 1-4 | yes | no | no |
| 2-3 | yes | no | no |
| E5 | yes | yes | yes |
| E8 | yes | yes | yes |
| B4 | yes | yes | yes |
| QAA1 | yes | yes | yes |
| QAA3 | yes | yes | yes |
| 2D5 | yes | no | no |
| 1C5 | yes | no | no |
| 346H-1 | yes | yes | yes |
| A3-2 | yes | yes | yes |
| 2C6 | yes | yes | no |
| K5D2 | yes | yes | yes |
| 155A-6 | yes | yes | no |
| 1D4 | no | no | no |
| 394H-5 | no | no | no |
| KAC4 | yes | yes | yes |
| K4D5 | yes | no | no |
| K4B6 | yes | yes | no |
| 1D6 | yes | yes | no |
| K5A3 | yes | yes | yes |
| A4-2 | yes | yes | yes |
| QDC4 | yes | yes | yes |

### Example 10: Mutant screening for amplification specificity to specific fragment

In order to screen out and obtain mutants with better amplification specificity, a specific gene hACTG1 was amplified with human genome as a template at lower annealing temperature. Under a limited polymerase concentration, each mutant was subject to amplification, to test it specificity performance according to the products, under the condition of lower annealing temperature. The primer sequences used were as follows:
hACTG1-F: GCTCAATGGGGTACTTCAGGGT (SEQ ID NO: 33);
hACTG1-R: GTGGACGTTACGTAAAAGGCCC (SEQ ID NO: 34).

Refer to Example 8 for amplification reaction procedure and system. The reaction products were detected by agarose gel electrophoresis. The mutant clones of chimeric polymerases based on wild type chimeric DNA polymerase and identified with amplification specificity are shown in Table 11, as examples.

**Table 10: Chimeric polymerase mutant clones screened out for amplification specificity**

| Clone name |
|---|
| 1-3 |
| 2-3 |
| E5 |
| E8 |
| B4 |
| QAA1 |
| QAA3 |
| 2D5 |
| 1C5 |
| A3-2 |
| 2C6 |
| K5D2 |
| 155A-6 |
| K4D5 |
| A4-2 |
| QDC4 |

The results of Examples 8-10 showed that the chimeric DNA polymerase, with further directed evolution, has further improved PCR performance such as salt tolerance, extension ability, sensitivity and/or amplification specificity, and the comprehensive performance of mutants E5, E8, A4-2, QDC4, QAA1 and QAA3 was particularly prominent. It was worth noting that these mutants were all further derived from mutant 1-4, indicating that the mutation combination or some mutations contained in mutant 1-4 plays a key functional role in displaying superior PCR performance. On the other hand, in addition to mutant 1-4 and derivative mutants thereof, mutant 1-3 also showed remarkable amplification sensitivity and specificity. The mutations contained in mutant 1-3 were integrated into derivative mutants of mutant 1-4 such as mutants 2D5, 1C5, 2C6 and K4D5, and most of them showed advantages in amplification specificity, indicating that mutation combination or some of the mutations contained in mutant 1-3 may play an important role in amplification specificity. In addition, similar to mutants E5, E8, A4-2, QDC4, QAA1 and QAA3, mutant A3-2 also showed outstanding comprehensive advantages in PCR performance, but such a mutation combination may not be conducive to transcription or translation of a target protein, and its expression level was low.

Reference throughout this specification to "an embodiment", "some embodiments", "one embodiment", "another example", "an example", "a specific example" or "some examples" means that a particular feature, structure, material, or characteristic described in connection with the embodiment or example is included in at least one embodiment or example of the present disclosure. Thus, the appearances of the phrases such as "in some embodiments", "in one embodiment", "in an embodiment", "in another example", "in an example", "in a specific example" or "in some examples", in various places throughout this specification are not necessarily referring to the same embodiment or example of the present disclosure. Furthermore, the particular features, structures, materials, or characteristics may be combined in any suitable manner in one or more embodiments or examples. Besides, any different embodiments and examples and any different characteristics of embodiments and examples may be combined by those skilled in the art without contradiction.

Although explanatory embodiments have been shown and described, it would be appreciated by those skilled in the art that the above embodiments cannot be construed to limit the present disclosure, and changes, alternatives, and modifications can be made in the embodiments in the scope of the present disclosure.

## Claims

1. A chimeric DNA polymerase, comprising:
a first peptide segment, having at least 80% homology with at least a first part of an amino acid sequence of a N-terminal domain of 9⁰ N DNA polymerase;
a second peptide segment, having at least 80% homology with at least a part of an amino acid sequence of an exonucleolytic domain of KOD DNA polymerase, wherein an N-terminal of the second peptide segment is connected with a C-terminal of the first peptide segment;
a third peptide segment, having at least 80% homology with at least a second part of the amino acid sequence of the N-terminal domain of 9⁰ N DNA polymerase, wherein an N-terminal of the third peptide segment is connected with a C-terminal of the second peptide segment;
a fourth peptide segment, having at least 80% homology with at least a first part of an amino acid sequence of a palm domain of KOD DNA polymerase, wherein an N-terminal of the fourth peptide segment is connected with a C-terminal of the third peptide segment;
a fifth peptide segment, having at least 80% homology with at least a part of an amino acid sequence of a finger domain of Pfu DNA polymerase, wherein an N-terminal of the fifth peptide segment is connected with a C-terminal of the fourth peptide segment;
a sixth peptide segment, having at least 80% homology with at least a second part of the amino acid sequence of the palm domain of KOD DNA polymerase, wherein an N-terminal of the sixth peptide segment is connected with a C-terminal of the fifth peptide segment; and
a seventh peptide segment, having at least 80% homology with at least a part of an amino acid sequence of a thumb domain of 9⁰ N DNA polymerase, wherein an N-terminal of the seventh peptide segment is connected with a C-terminal of the sixth peptide segment.

2. The chimeric DNA polymerase according to claim 1, wherein the first peptide segment has at least 80% homology with an amino acid sequence at positions 1 to 390 of the amino acid sequence for 9⁰ N DNA polymerase.

3. The chimeric DNA polymerase according to claim 1, wherein the second peptide segment has at least 80% homology with an amino acid sequence at positions 391 to 1014 of the amino acid sequence for KOD DNA polymerase.

4. The chimeric DNA polymerase according to claim 1, wherein the third peptide segment has at least 80% homology with an amino acid sequence at positions 1015 to 1116 of the amino acid sequence for 9⁰ N DNA polymerase.

5. The chimeric DNA polymerase according to claim 1, wherein the fourth peptide segment has at least 80% homology with an amino acid sequence at positions 1117 to 1341 of the amino acid sequence for KOD DNA polymerase.

6. The chimeric DNA polymerase according to claim 1, wherein the fifth peptide segment has at least 80% homology with an amino acid sequence at positions 1345 to 1500 of the amino acid sequence for Pfu DNA polymerase.

7. The chimeric DNA polymerase according to claim 1, wherein the sixth peptide segment has at least 80% homology with an amino acid sequence at positions 1498 to 1770 of the amino acid sequence for KOD DNA polymerase.

8. The chimeric DNA polymerase according to claim 1, wherein the seventh peptide has at least 80% homology with an amino acid sequence at positions 1771 to 2328 of the amino acid sequence for 9⁰ N DNA polymerase.

9. The chimeric DNA polymerase according to claim 1, wherein the chimeric DNA polymerase is of an amino acid sequence as depicted in SEQ ID NO: 1.

10. The chimeric DNA polymerase according to claim 9, wherein the chimeric DNA polymerase has at least one mutation selected from the following mutations, compared with the amino acid sequence as depicted in SEQ ID NO: 1:
M162I, I540V, A598T, H728Q, F37Y, D48V, R100H, Y221N, K243N, Q245L, I271T, E296V, N307S, F751Y, L758Q, V766I, E154A, L44Q, Y149H, R196C, F217H, D346H, D715E, F155A, Q94H and Q94L.

11. The chimeric DNA polymerase according to claim 9, wherein the chimeric DNA polymerase has a group of mutations selected from the following groups:
group I: M162I, I540V, A598T and H728Q;
group II: F37Y, D48V, R100H, Y221N, K243N, Q245L, I271T, E296V, N307S, F751Y, L758Q, V766I and E154A;
group III: F37Y, L44Q, D48V, R100H, Y149H, K243N, Q245L, I271T, E296V, N307S, F751Y, L758Q, V766I and E154A;
group IV: F37Y, D48V, R100H, R196C, F217H, Y221N, K243N, Q245L, I271T, E296V, N307S, D346H, F751Y, L758Q, V766I and E154A;
group V: F37Y, D48V, Q94L, R100H, Y221N, K243N, Q245L, I271T, E296V, N307S, F751Y, L758Q, V766I and E154A;
group VI: E296V, N307S, F751Y, L758Q and E154A;
group VII: F37Y, D48V, Q94H, R100H, Y221N, K243N, Q245L, I271T, E296V, N307S, D715E, H728Q, F751Y, L758Q, V766I and E154A; and
group VIII: F37Y, D48V, Q94L, R100H, F155A, Y221N, K243N, Q245L, I271T, E296V, N307S, F751Y, L758Q, V766I and E154A.

12. The chimeric DNA polymerase according to claim 1, wherein the chimeric DNA polymerase is of an amino acid sequence as depicted in any one of SEQ ID NOs: 2-9.

13. An isolated nucleic acid, encoding a chimeric DNA polymerase according to any one of claims 1 to 9.

14. The isolated nucleic acid according to claim 13, wherein the isolated nucleic acid is of a nucleotide sequence as depicted in any one of SEQ ID NOs: 10-18.

15. A construct, comprising an isolated nucleic acid according to any one of claims 13 to 14.

16. A recombinant cell or a recombinant microorganism, comprising an isolated nucleic acid according to any one of claims 13 to 14.

17. A method for obtaining a chimeric DNA polymerase according to any one of claims 1 to 12, comprising:
cultivating a recombinant cell or a recombinant microorganism according to claim 16 in a condition suitable for expressing the chimeric DNA polymerase, so as to obtain the chimeric DNA polymerase.

18. A kit, comprising a chimeric DNA polymerase of any one of claims 1 to 12, an isolated nucleic acid of any one of claims 13 to 14, a construct of claim 15, or a recombinant cell or a recombinant microorganism of claim 16.

19. Use of a chimeric DNA polymerase of any one of claims 1 to 12, an isolated nucleic acid of any one of claims 13 to 14, a construct of claim 15, a recombinant cell or a microorganism of claim 16, or a kit of claim 18 for DNA amplification.

20. The use according to claim 19, wherein the chimeric DNA polymerase, the isolated nucleic acid, the construct, the recombinant cell or the recombinant microorganism, or the kit is used for gene screening, sequencing or mutation detection.
